# EUROPEAN PATENT APPLICATION

(11) **EP 4 659 810 A1**
(43) Date of publication of application: **10.12.2025**
(21) Application number: 24180876.5
(22) Date of filing: 07.06.2024
(51) Int. Cl.: A61P 31/20, C07K 16/08

(54) **BKV NEUTRALIZING ANTIBODIES**

(71) Applicant: Université de Strasbourg, 67000 Strasbourg (FR); Institut National de la Santé et de la Recherche Médicale, 75013 Paris (FR); Centre National de la Recherche Scientifique, 75016 Paris (FR); LES HOPITAUX UNIVERSITAIRES DE STRASBOURG, 67000 Strasbourg (FR); Université Grenoble Alpes, 38400 Saint-Martin-d'Hères (FR); Commissariat à l'Energie Atomique et aux Energies Alternatives, 75015 Paris (FR); Centre Hospitalier Universitaire Grenoble Alpes, 38043 Grenoble Cedex 9 (FR)
(72) Inventor: POIGNARD, Pascal, 38044 GRENOBLE (FR); DERGAN-DYLON, Leonardo Sebastián, 38044 GRENOBLE (FR); BUISSON, Marlyse, 38044 GRENOBLE (FR); SCHOEHN, Guy, 38044 GRENOBLE (FR); PEDENKO, Borys, 38044 GRENOBLE (FR); FAFI-KREMER, Samira, 67000 STRASBOURG (FR); SOLIS, Morgane, 67000 STRASBOURG (FR); CAILLARD-OHLMANN, Sophie, 67000 STRASBOURG (FR)
(74) Representative: Ipsilon

(57) **Abstract**

The present patent application relates to the fields of antibodies and polyomaviruses. More specifically, the present patent application relates to a human monoclonal antibody, or antigen-binding fragment thereof, directed against a polyomavirus, in particular BKV, and that is useful in the treatment of an infection by said polyomavirus or associated-disorder thereof.

## Description

### INTRODUCTION

The present patent application relates to the fields of antibodies and polyomaviruses. More specifically, the present patent application relates to a human monoclonal antibody, or antigen-binding fragment thereof, directed against a polyomavirus, in particular BKV, and that is useful in the treatment of an infection by said polyomavirus or associated-disorder thereof.

Human polyomaviruses, such as BK virus (BKV) or JC virus (JCV), are responsible for a common childhood infection highly prevalent worldwide, which elicits a neutralizing antibody and cellular immune response, while establishing a dormant and persistent infection in the kidney with minimal clinical manifestations. Despite its minimal impact among healthy individuals, polyomaviruses can reactivate and can cause severe pathology in transplant recipients and other immunocompromised individuals. It is estimated that up to 30% of the 100,000 kidney transplants performed annually worldwide are potentially compromised by BKV replication or, in its progressive form, by BKV-associated nephropathy (BKVAN) (Barth et al., Crit Rev Microbiol. 2017; 43: 178-195; Dharnidharka et al., Transplantation 2009; 87: 1019-1026). Early BKV replication after transplantation increases the risk of late acute rejection, which can expose recipients to graft loss when BKVAN develops (Seifert et al., J Am Soc Nephrol. 2017; 28: 1314-1325). Prolonged BKV replication also increases the risk of urothelial carcinoma in kidney transplant recipients (Gupta et al., Am J Transplant. 2018; 18(1): 245-252). In addition, BKV infection has been reported to cause symptomatic haemorrhagic cystitis in approximately 5% of the 50,000 hematopoietic stem cell transplants performed annually worldwide, as well as in 5% of cancer patients treated with high dose cyclophosphamide (Barth et al., Crit Rev Microbiol. 2017; 43: 178-195; Dharnidharka et al., Transplantation 2009; 87: 1019-1026), which leads to prolonged hospitalization and, in severe cases, can be fatal. As for the related JCV, its reactivation can typically impact the central nervous system (CNS), which can result in progressive multifocal leukoencephalopathy (PML), a condition that is also frequently fatal.

Current guidelines recommend regular screening to detect BKV viruria or viremia after transplantation. Patients exhibiting persistent high BKV DNA loads usually undergo an immunosuppression reduction to lessen BKV replication and prevent BKVAN occurrence. However, because of its delayed nature, this pre-emptive strategy is not always successful and can actually increase the risk of graft rejection and death (Seifert et al., J Am Soc Nephrol. 2017; 28: 1314-1325; J Am Soc Nephrol. 2015; 26(4): 966-75). Besides, some individuals who are infected with one BKV subtype may remain vulnerable to other BKV subtypes after implementation of T cell immunosuppression, due to the absence of protective cross-reactive antibody responses.

There are at present no available BKV (or JCV)-specific antiviral therapies on the market.

Human intravenous immunoglobulin preparations (i.v. Ig, also referred as IVIG) have previously been shown to be effective in the prevention and treatment of many human viral infections in transplant patients. Randhawa et al. demonstrated that IVIG preparations commercialized under the names Privigen^{®} and Cytogam^{®} (both from CSL Behring GmbH, Marburg, Germany) contain antibodies capable of neutralizing all major BKPV genotypes *in vitro* (Am J Transplant. 2015; 15: 1014-1020), albeit much less efficiently for genotype IV. These preparations however contain a mixture of uncharacterized human IgGs that are pooled from human plasma; they are thus susceptible to batch-to-batch variations and are not specific to BKV. Some case reports even mentioned the presence of anti-blood group antibodies (ant-A or anti-B) in commercial IVIG products, which raises the risk of hemolysis in non-group O recipient patients.

Given the incomplete success of pre-emptive and supportive strategies for BKV-associated diseases, there is thus an urgent need to develop new anti-BKV preventive and therapeutic strategies, that are specific, safe, and highly effective against a wide range of BKV subtypes, preferably also against JCV.

The present invention addresses the above discussed need in the art.

### SUMMARY OF THE INVENTION

The present patent invention is based on the development of novel human monoclonal antibodies, or antigen-binding fragments thereof, capable of binding and neutralizing BKV virus, with desirable pharmacokinetic properties and other desirable attributes, including binding and neutralizing of BKV subtype I, II and IV, as well as binding and neutralizing of JCV. Targeted therapy with those antibodies can accordingly provide a novel therapeutic approach for BKV and JCV infections and address the large unmet medical needs associated with JCV and BKV related diseases.

The development of those antibodies is based on a novel approach which enabled the selection of a particular PBMCs (peripheral blood mononuclear cells) human donor with high anti-BKV antibody neutralization titer in serum, across various BKV subtypes. Memory B cells of this elite BKV neutralizer were then isolated for rescue of those monoclonal antibodies. Out of the 53 monoclonal antibodies isolated from this donor, 6 were selected for their high affinity and broad neutralization towards BKV, and their epitopes characterized. Interestingly, these antibodies were able to further neutralize JCV, and lacked auto- and poly-reactivity, thereby confirming their clinical suitability.

Accordingly, in a first aspect, the present invention relates to a monoclonal antibody, preferably a human monoclonal antibody, or antigen-binding fragment or chain thereof, that binds and neutralizes a plurality of BK virus subtypes including at least subtypes I, II and IV, said antibody having a combination of a heavy chain variable domain (VH) and/or a light chain variable domain (VL) with the six following complementary-determining regions (CDRs):
- a VH-CDR1 comprising, or consisting of, amino acid sequence GFX₁X₂RX₃YX₄ (SEQ ID NO: 1), wherein X₁ is S, T or N, preferably is S; X₂ is F, I or L, preferably is F or I; X₃ is S or G; and X₄ is A or G, preferably is A;
- a VH-CDR2 comprising, or consisting of, amino acid sequence LX₅SX₆GX₇X₈X₉, wherein X₅ is N, T, S or R, preferably is N or T; X₆ is A, S or N, preferably is A or N; X₇ is V, A or T, preferably is V or A; X₈ is S or T; X₉ is T or A, preferably is T;
- a VH-CDR3 comprising, or consisting of, amino acid sequence AX₁₀DRX₁₁X₁₂X₁₃WLGSEPX₁₄DP (SEQ ID NO: 2), wherein X₁₀ is K or R, preferably is K; X₁₁ is G, D or N, preferably is G or D; X₁₂ is V or A; X₁₃ is E or Q, preferably is E; X₁₄ is I, F or L, preferably is I or F;
- a VL-CDR1 comprising, or consisting of, amino acid sequence QX₁₅X₁₆X₁₇X₁₈X₁₉Y, wherein X₁₅ is R or S, preferably is R; X₁₆ is VorL, preferably is V; X₁₇ is S or G, preferably is S; X₁₈ is S or N, preferably is S; X₁₉ is N or S;
- a VL-CDR2 comprising, or consisting of, amino acid sequence X₂₀AS, wherein X₂₀ is R, G or D, preferably is R or G; and
- a VL-CDR3 comprising, or consisting of, amino acid sequence QQYGSX₂₁PRS (SEQ ID NO: 3), wherein X₂₁ is S or P, preferably is S.

In a preferred embodiment of the antibody, or antigen-binding fragment thereof, according to invention:
- the VH-CDR1 comprises, or consists of, amino acid sequence SEQ ID NO: 6, 13, 19, 25, 28 or 34;
- the VH-CDR2 comprises, or consists of, amino acid sequence SEQ ID NO: 7, 14, 20, 29 or 35;
- the VH-CDR3 comprises, or consists of, amino acid sequence SEQ ID NO: 8, 15, 30 or 36;
- the VL-CDR1 comprises, or consists of, amino acid sequence SEQ ID NO: 9, 16, 21, 31 or 37;
- the VL-CDR2 comprises, or consists of, amino acid sequence RAS, GAS, or DAS; and
- the VL-CDR3 comprises, or consists of, amino acid sequence SEQ ID NO: 10 or 22.

In a preferred embodiment, the six complementary determining regions (CDRs) of the antibody or antigen-binding fragment according to the invention are selected from either of the following combinations:
(a) VH-CDR1, VH-CDR2 and VH-CDR3 comprising, or consisting of, amino acid sequence SEQ ID NO: 6, 7 and 8, respectively; and VL-CDR1, VL-CDR2 and VL-CDR3 comprising, or consisting of, amino acid sequence SEQ ID NO: 9, RAS and SEQ ID NO: 10, respectively; or
(b) VH-CDR1, VH-CDR2 and VH-CDR3 comprising, or consisting of, amino acid sequence SEQ ID NO: 13, 14 and 15, respectively; and VL-CDR1, VL-CDR2 and VL-CDR3 comprising, or consisting of, amino acid sequence SEQ ID NO: 16, GAS and SEQ ID NO: 10, respectively; or
(c) VH-CDR1, VH-CDR2 and VH-CDR3 comprising, or consisting of, amino acid sequence SEQ ID NO: 19, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(d) VH-CDR1, VH-CDR2 and VH-CDR3 comprising, or consisting of, amino acid sequence SEQ ID NO: 25, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(e) VH-CDR1, VH-CDR2 and VH-CDR3 comprising, or consisting of, amino acid sequence SEQ ID NO: 28, 29 and 30, respectively; and VL-CDR1, VL-CDR2 and VL-CDR3 comprising, or consisting of, amino acid sequence SEQ ID NO: 31, GAS and SEQ ID NO: 10, respectively; or
(f) VH-CDR1, VH-CDR2 and VH-CDR3 comprising, or consisting of, amino acid sequence SEQ ID NO: 34, 35 and 36, respectively; and VL-CDR1, VL-CDR2 and VL-CDR3 comprising, or consisting of, amino acid sequence SEQ ID NO: 37, GAS and SEQ ID NO: 10, respectively.

In a preferred embodiment, the variable domains of the antibody or antigen-binding fragment thereof according to the invention are as follows:
- the heavy chain variable domain (VH) comprises, or consists of, an amino acid sequence having at least 80%, 85%, 90%, 91%, 92%, 93%, 94%, 95%, 96%, 97%, 98% or 99% sequence identity to: 
EVQLLESGGX22LVQPGGSLRLSCX23ASGFX1X2RX3YX4MNWVRQAPGX24GLE
WVSSLX5SX6GX7X8X9YYAX25SVKGRFTISRDNX26KNTX27X28LQMX29SLRGEDT
AVYYCAX10DRX11X12X13WLGSEPX14DPWGQGTX30VTVSS (SEQ ID NO: 4),
 wherein X₁ is S, T or N, preferably is S; X₂ is F, I or L, preferably is F or I; X₃ is S or G; and X₄ is A or G, preferably is A; X₅ is N, T, S or R, preferably is N or T; X₆ is A, S or N, preferably is A or N; X₇ is V, A or T, preferably is V or A; X₈ is S or T; X₉ is T or A, preferably is T; X₁₀ is K or R, preferably is K; X₁₁ is G, D or N, preferably is G or D; X₁₂ is V or A; X₁₃ is E or Q, preferably is E; X₁₄ is I, F or L, preferably is I or F; X₂₂ is G or A, preferably is G; X₂₃ is A or V; X₂₄ is K or R, preferably is K; X₂₅ is E or D; X₂₆ is S or F, preferably is S; X₂₇ is L or M, preferably is L; X₂₈ is Y or F; X₂₉ is N, H or D, preferably is N or H; and X₃₀ is L or M, preferably is L; and
- the light chain variable domain (VL) comprises, or consists of, an amino acid sequence having at least 80%, 85%, 90%, 91%, 92%, 93%, 94%, 95%, 96%, 97%, 98% or 99% sequence identity to: EIVLTQSPGTLSLSPGERATLSCRASQX₁₅X₁₆X₁₇X₁₈X₁₉YLAWYQHKX₃₁GX₃₂APR LLIYX₂₀ASX₃₃RAX₃₄GIPDRFSGSX₃₅SGTDTLTISRLEPEDFAVYYCQQYGSX₂₁PR SFGQGTKLEIK (SEQ ID NO: 5), wherein X₁₅ is R or S, preferably is R; X₁₆ is V or L, preferably is V; X₁₇ is S or G, preferably is S; X₁₈ is S or N, preferably is S; X₁₉ is N or S; X₂₀ is R, G or D, preferably is R or G; X₂₁ is S or P, preferably is S; X₃₁ is P or F; X₃₂ is Q or L, preferably is Q; X₃₃ is S or R; X₃₄ is T or I, preferably is T; and X₃₅ is G or E, preferably is G;
with the proviso that the CDRs are as described herein.

In a preferred embodiment of the antibody, or antigen-binding fragment thereof, according to invention:
- the VH comprises, or consists of, an amino acid sequence having at least 80% sequence identity to any one of SEQ ID NO: 11, 17, 23, 26, 32 or 38, preferably SEQ ID NO: 11 or 17; and
- the VL comprises, or consists of, an amino acid sequence having at least 80% sequence identity to any one of SEQ ID NO: 12, 18, 24, 27, 33 or 39, preferably SEQ ID NO: 12 or 18;
with the proviso that the CDRs are as described herein.

In a preferred embodiment, the VH and the VL of the antibody, or antigen-binding fragment thereof, according to invention are selected from any one of the following combinations:
(i) VH having at least 80% sequence identity to SEQ ID NO: 11 and VL having at least 80% sequence identity to SEQ ID NO: 12, with the proviso that the CDRs thereof are as described herein; or
(ii) VH having at least 80% sequence identity to SEQ ID NO: 17 and VL having at least 80% sequence identity to SEQ ID NO: 18, with the proviso that the CDRs thereof are as described herein; or
(iii) VH having at least 80% sequence identity to SEQ ID NO: 23 and VL having at least 80% sequence identity to SEQ ID NO: 24, with the proviso that the CDRs thereof are as described herein; or
(iv) VH having at least 80% sequence identity to SEQ ID NO: 26 and VL having at least 80% sequence identity to SEQ ID NO: 27, with the proviso that the CDRs thereof are as described herein; or
(v) VH having at least 80% sequence identity to SEQ ID NO: 32 and VL having at least 80% sequence identity to SEQ ID NO: 33, with the proviso that the CDRs thereof are as described herein; or
(vi) VH having at least 80% sequence identity to SEQ ID NO: 38 and VL having at least 80% sequence identity to SEQ ID NO: 39, with the proviso that the CDRs thereof are as described herein.

In a preferred embodiment, the antibody, or antigen-binding fragment thereof, according to invention further binds and neutralizes JC virus (JCV).

In a further aspect, the invention pertains to an antibody, or antigen-binding fragment thereof, that binds and neutralizes a plurality of BK virus subtypes including at least subtype I, II and IV, wherein said antibody has one or more of the following characteristics:
(1) competes for binding to a BKV viral protein 1 (VP1), with a reference antibody as described herein, such as antibody E3P7 (VH: SEQ ID NO: 11; VL: SEQ ID NO: 12), D10P4 (VH: SEQ ID NO: 17; VL: SEQ ID NO: 18), D6P7 (VH: SEQ ID NO: 23; VL: SEQ ID NO: 24), E4P7(VH: SEQ ID NO: 26; VL: SEQ ID NO: 27), G5P4 (VH: SEQ ID NO: 32; VL: SEQ ID NO: 33), and/or B8P4 (VH: SEQ ID NO: 38; VL: SEQ ID NO: 39); and/or
(2) binds to an epitope comprising one or more amino acids selected from P59, D60, E61, L63, F66, L68, K69, N74, D75, F76, S78, E82, H139, F271, T277 and/or S275 in a BKV viral protein 1 (VP1) of SEQ ID NO: 40; and/or
(3) has reduced binding or loses binding to a mutant BKV viral protein 1 (VP1), said mutant containing one or more of the following amino acid mutations in SEQ ID NO: 40: P59A, D60A, E61A, L63A, F66A, L68A, K69A, N74A, D75A, F76A, S78A, E82A, H139A, F271A, T277A, and/or S275A.

In a preferred embodiment, the antibody, or antigen-binding fragment thereof, according to the invention, is an IgG, an IgA, an sIgA, an IgM or a nanobody, preferably is an IgG, said IgG being preferably an IgG1, IgG2, IgG3 or IgG4.

In a preferred embodiment, the antibody, or antigen-binding fragment thereof, according to the invention, further comprises, especially in the Fc region, modifications which modulate Fc/FcRn binding, complement binding, complement-dependent cytotoxicity (CDC), antibody-dependent cellular toxicity (ADCC), antibody dependent cell phagocytosis (ADCP), glycosylation and/or pharmacokinetics such as half-life.

In a preferred embodiment, the antibody, or antigen-binding fragment thereof, according to the invention, comprises one or more non-natural amino acids.

In a preferred embodiment, the antibody, or antigen-binding fragment thereof, according to the invention, is comprised in an immunoconjugate, such as an immunoconjugate wherein the antibody, or antigen-binding fragment thereof, is conjugated with another moiety, such as another moiety selected from another antibody, including another anti-BKV antibody, a cytotoxic moiety (to form an ADC), a cell-penetrating compound or a tissue-penetrating compound.

In a preferred embodiment, the antibody, or antigen-binding fragment thereof, according to the invention, is labeled with a detectable molecule.

In another aspect, the invention is directed to a combination of two or more antibodies, or antigen-binding fragments thereof, as described herein.

In another aspect, the invention relates to a set of nucleic acids encoding the antibody, or antigen-binding fragment thereof, according to the invention, or to a vector comprising said set of nucleic acids.

In another aspect, the invention relates to a host cell expressing the antibody, or antigen-binding fragment thereof according to the invention, or comprising the set of nucleic acids or the vector according to the invention.

In another aspect, the invention pertains to a pharmaceutical or diagnostic composition comprising: (1) at least one of the antibody, or antigen-binding fragment thereof, according to the invention, or any combination thereof; and (2) optionally at least one pharmaceutically or diagnostically acceptable excipient.

In a preferred embodiment, the pharmaceutical composition is in a form suitable for a sustainable release, and/or for intravenous, intramuscular, subcutaneous, intranasal, or intrathecal administration, or for administration by infusion or by aerosol.

In a further aspect, the invention is directed to a pharmaceutical composition as described herein, for use as a medicament.

In a preferred embodiment, the pharmaceutical composition is for use in the treatment of a BK virus (BKV) infection or associated disorder thereof, in a subject in need thereof.

In a preferred embodiment, the subject is an immunocompromised subject, especially a graft-recipient such as a kidney-graft or bone-marrow-graft recipient.

In a preferred embodiment, the disorder associated with a BK virus (BKV) infection is a BKV-related leukodystrophy.

In another aspect, the invention relates to a pharmaceutical composition as described herein and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus.

In another aspect, the invention pertains to two or more antibodies or antigen-binding fragments according to the invention, as a combined preparation for simultaneous, separate or sequential use as a medicament, preferably in the treatment of a BK virus (BKV) infection or associated disorder thereof, in a subject in need thereof.

In a further aspect, the invention relates to an *in vitro* use of a diagnostic composition according to the invention, for detecting the presence of BKV in a sample, preferably in a biological sample.

In another aspect, the invention is directed to an *in vitro* use of an anti-idiotypic antibody that binds to the antibody or antigen-binding fragment thereof according to the invention, for measuring the concentration of said antibody or fragment in a sample, preferably in a biological sample.

### LEGENDS TO THE FIGURES

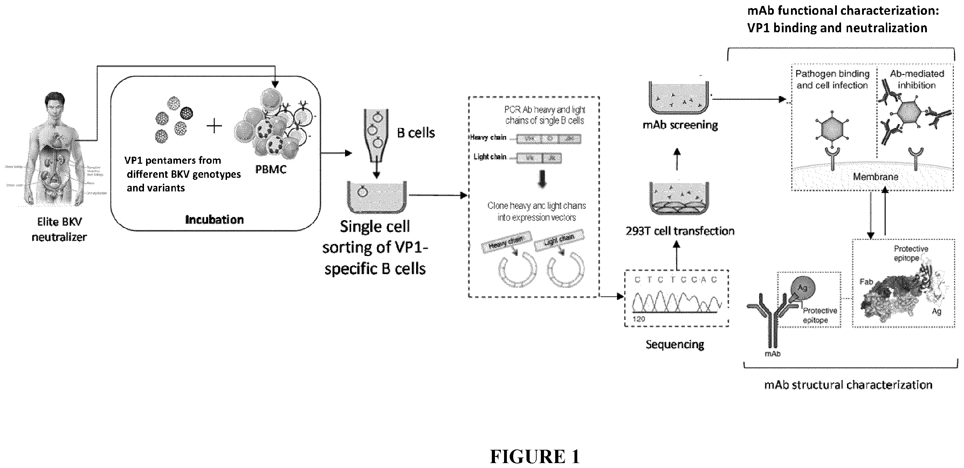

**Figure 1****.** Workflow representation of the generation of monoclonal antibodies by analysis of the memory B-cell repertoire of elite BKV neutralizers.
**Figure 2****.** Cryo-EM structure of BKV VP1 pentamers complexed with Fabs. The interfacing area is based on the occupancy by the heavy and light chains.
**Figure 3****.** Epitope mapping by cryo-EM. **(A)** Interactions of Fab D10P4 with VP1 pentamers. **(B)** Interactions of Fab E3P7 with VP1 pentamers.
**Figure 4****.** Antibody blockade binding of VP1 pentamer to gangliosides (alpha 2-3 glycan) on HEK293 cells.

### DETAILED DESCRIPTION OF THE INVENTION

The present invention may be understood more readily by reference to the following detailed description, including preferred embodiments of the invention and examples described below.

### Definitions

Unless otherwise defined herein or required by context, terms used in connection with the present invention, such as scientific and technical terms, shall have the meanings that are commonly known and understood in the art. Additional definitions may be provided throughout the detailed description.

The term "*antibody*" as used herein refers to a polypeptide of the immunoglobulin family that is capable of specific binding to a given antigen (herein, the Viral Protein 1, or VP1 of the BK or JC virus). Basic immunoglobulin structures in vertebrate systems are well understood (Harlow et al., Antibodies: A Laboratory Manual, Cold Spring Harbor Laboratory Press, ed. 1988). For example, antibodies that are naturally-occurring in humans and many mammals comprise four polypeptides - two full-length light chains (abbreviated herein as LC) and two full-length heavy chains (abbreviated herein as HC) - which are joined to one another with disulfide bonds to form a Y-shaped protein. Each of those chains, whether light or heavy, contains a "*constant*" domain (or region), as well as a "*variable*" domain (or region): constant domains usually confer important biological properties such as secretion, transplacental mobility, Fc receptor binding, complement binding, and the like; while variable domains determine antigen recognition and specificity. Because these variable domains are essential for antigen binding, it will be appreciated that an antibody as disclosed herein comprises at least the variable domain of a heavy chain (abbreviated herein as VH) and at least the variable domain of a light chain (abbreviated herein as VL). These VH and VL domains can be further subdivided into regions of hypervariability, termed complementarity determining regions (CDR), which are interspersed with regions that are more conserved, termed framework regions (FR). Each VH and VL of an antibody is typically composed of three CDRs and four FRs arranged (i.e. operably linked) from N- to C-terminus in the following preferred order: FR1, CDR1, FR2, CDR2, FR3, CDR3, and FR4. This is these regions of hypervariability of the heavy and light chains, especially the CDRs, that contain a binding domain (paratope) that interacts with a particular region of the antigen (epitope). As for the constant domains, the heavy chain may contain three constant domains, referred as CH1, CH2 and CH3, while the light chain may contain one constant domain, referred as CL. The two constant domains CH2 and CH3 of the heavy chains essentially form the Fc region (the trunk of the Y shape), which is capable of modulating immune cell activity once the antibody binds to the antigen. Antibodies are typically divided into five main immunoglobulin isotypes or classes (e.g., IgG, IgE, IgM, IgD, IgA and IgY), which are themselves divided into discrete subclasses (e.g., IgG1, IgG2, IgG3, IgG4, IgA1 and IgA2). This classification is based on the nature of heavy chain they contain, which includes alpha, delta, epsilon, gamma, and mu heavy chains, and essentially defines the function triggered by the antibody. In IgG, which make up the vast majority of antibodies in humans, heavy chains are as described above (VH and CH1, CH2, CH3). IgA and IgD also have three constant domains per heavy chain, whereas IgM and IgE each have four constant domains per heavy chain. IgG are herein particularly preferred. Antibody light chains can be classified in mammals either as kappa light chains or lambda light chains. The term antibody also encompasses, without limitation, antibodies that are polyclonal or monoclonal, human/ humanized or non-human/non-humanized, chimeric or non-chimeric.

An "*epitope*" or "*antigenic determinant*" refers herein to a region of an antigen to which an antibody binds to. The particular domain of the antibody which binds an epitope is named "*paratope*"*.* Epitopes of a protein antigen can be formed from contiguous amino acids (i.e. it is a linear epitope) or noncontiguous amino acids that are juxtaposed by tertiary folding of the antigen (i.e. it is a conformational epitope). Linear epitopes are typically retained when exposed to denaturing solvents whereas conformational epitopes are typically lost on treatment with denaturing solvents. An epitope can typically include at least 3, preferably at least 4, 5, 6, 7, 8, 9 or 10 amino acids in a unique spatial conformation. Methods for determining spatial conformation of epitopes include, for example, x-ray crystallography, cryo-electron microscopy (cryo-EM), or nuclear magnetic resonance (NMR). Epitope mapping, which aims at identifying the specific amino acids of the antigen involved in the binding with an antibody, can be determined e.g. by array-based oligo-peptide scanning, site-directed mutagenesis mapping, high-throughput shotgun mutagenesis epitope mapping, or cross-linking-coupled mass spectrometry (See e.g. Epitope Mapping Protocols, Methods in Molecular Biology, Vol. 66, Glenn E. Morris, Ed. 1996). One specific example of mutagenesis mapping relies on the arginine/glutamic scanning protocol: briefly, amino acids at all or some specific positions of the antigen are substituted with either arginine or glutamic acid (or arginine with glutamic acid and vice-versa); binding to the mutants is then assessed to determine which residues affect binding. A similar approach relies on alanine scanning; substitution with alanine indeed eliminates side-chain interactions without altering main-chain conformation or introducing steric or electrostatic effects. Antibodies that recognize the same epitope can be identified in a simple immunoassay showing the ability of one antibody to block the binding of another antibody to a target antigen.

By contrast to polyclonal antibodies which recognize different epitopes of the same antigen, "*monoclonal antibodies*" or "*mAbs*" are antibodies which recognize a unique epitope on an antigen. That is because monoclonal antibodies typically derive from a single parent cell, such as a single B cell parent clone (i.e. single cell lineage), while polyclonal antibodies would stem from different parent cells, such as different B cell parent clones (i.e. from a different cell lineage).

A "*human antibody*" refers herein to an antibody, as defined above, that is naturally occurring in a human or that is produced in a suitable host cell, such as a human cell, as long as it contains antibody amino acid sequences that are characteristics of the human species. By contrast, humanized antibodies originate from a non-human species and have been modified, especially in the framework regions and/or constant regions, to resemble human antibodies so as to avoid any undesired immunogenic reactions when administered to humans.

The term "*antigen-binding fragment*", "*antibody functional fragment*" or "*antibody fragment*" designates herein an immunologically active fragment or portion of an antibody as defined above. This means that said fragment retains the same, or substantially the same, ability to bind to the same the antigen, more particularly to the same epitope, as the antibody from which it is derived. To do so, the antigen-binding fragment will typically retain at least the antigen-binding region of a traditional four-chain antibody, such as the CDR(s). Examples of antigen-binding fragments are well-known in the art, and include, to name a few, Fab (i.e. fragment antigen-binding region, which is comprised of one constant and one variable domain of each of the heavy chain and light chain i.e. VL, CL, VH and CH1), F(ab') (corresponding to a Fab, on which a few amino acids have been added at the C-terminus of the CH1 domain of the heavy chain, including one or more cysteines from the hinge region), F(ab')2 (corresponding to two Fabs, or a bivalent Fab, connected at a hinge region), scFv (single chain variable fragment, which is comprised of a VL and a VH), or HCAb (heavy chain antibody, which is comprised of a single heavy chain with one variable domain (VHH)). Antigen-binding fragments may also include single domain antibodies, maxibodies, minibodies, nanobodies, intrabodies, diabodies, triabodies, tetrabodies, or v-NAR. If needed, an Fc region may be added to the antigen-binding fragment.

As used herein, the terms "*Fc region*"*,* "*Fc domain*" and "*fragment crystallizable region*" are interchangeable and refer to the tail region of an antibody that interacts with cell surface receptors called Fc receptors. The Fc region is typically composed of two domains, optionally identical, derived from the second and third constant domains of the antibody two heavy chains (i.e., CH2 and CH3 domains). A portion of the Fc region may refer to the CH2 or the CH3 domain. Optionally, the Fc region may further comprise all or a portion of the antibody hinge region, which is the flexible region generally comprised between the CH1 and CH2 domains. Accordingly, the Fc domain may comprise the hinge, the CH2 domain and the CH3 domain. Optionally, the Fc domain is that from IgG1, IgG2, IgG3 or IgG4, optionally with IgG1 hinge-CH2-CH3 or IgG4 hinge-CH2-CH3.

As explained above, each VH and VL of an antibody typically comprises regions of hypervariability termed "*complementarity determining regions*"*,* "*complementarity determining domains*" or "*CDRs*"*.* The CDRs are the antigen-binding site of the antibody variable domains that harbors specificity for and confer binding affinity to said antigen. In other words, the CDRs are structurally complementary to the epitope of the antigen and are thus directly responsible for the binding specificity. There are typically three CDRs (CDR1 to 3, numbered sequentially starting from the N-terminus) in each human VH or VL, constituting in total about 15 to 20% of the variable domain therein. This means that a conventional antibody generally contains six CDRs. These CDRs can be referred to by their region and sequential order. For example, "*VH-CDR1*" refers to the first CDR of the heavy chain variable domain; *"VH-CDR2* " to the second CDR of this domain; "*VH-CDR3* " to the third CDR of this domain. Likewise, "*VL-CDR1* " refers to the first CDR of the light chain variable domain; "*VL-CDR2* " to the second CDR of this domain; "*VL-CDR3*" to the third CDR of this domain. Given the particular structure of an antibody, their CDRs are not contiguous to each other.

The term "*framework regions*" or "*FRs*" refer to regions of the VH or VL that are interposed between the CDRs of an antibody. In general, these regions act as a scaffold that provides for positioning the CDRs in correct orientation, so as to support the binding of these CDRs to its cognate epitope. Framework regions are also known to exhibit far less variation in amino acid sequence than CDRs (Kuby, Immunology, 4th ed., Chapter 4. W.H. Freeman & Co., New York, 2000), and as such, are relatively conserved within a species, especially within an immunoglobulin class /subclass. Mutations of framework regions may nevertheless occur in cells and/or during affinity maturing of an antibody; such mutations are encompassed in the scope of the present invention as long as the antibody, or antigen-binding fragment thereof, retains at least its ability to bind its antigen. There are typically 4 FRs (FR1 to 4, numbered sequentially starting from the N-terminus) in each human VH or VL, constituting in total about 80 to 85 % of the variable domain therein. The framework regions of an antibody heavy chain may be referred to as "*HFRF*"*,* "*HFR2*"*,* "*HFR3*" and "*HFR4*"*,* while the framework regions of an antibody light chain may be referred to as "*LFRI*"*,* "*LFR2*"*,* "*LFR3*" and "*LFR4*"*.*

The precise amino acid sequence boundaries of each antibody region or domain can be readily determined using any of a number of techniques and/or schemes that are well-known in the art. Examples of numbering schemes include the EU numbering scheme (Edelman et al. Proc. Natl. Acad. Sci. USA 1969; 63; 78-85), Kabat numbering scheme (Kabat et al., Sequences of Proteins of Immunological Interest, NIH Publication No. 91-3242, 5th ed. 1991), Chothia numbering scheme (Al-Lazikani et al., J. Mol. Biol. 1997; 273:927-948), Contact numbering scheme (MacCallum et al., J. Mol. Biol. 1996; 262:732-745), IMGT numbering scheme (Lefranc et al., Immunol Today. 1997; 18(11):509 ; Lefranc et al., Dev. Comp. Immunol. 2003; 27:55-77), AHo numbering scheme (Honegge and Pluckthun, J. Mol. Biol. 2001; 309:657-70), Martin numbering scheme (enhanced Chotia or AbM; Abhinandan et al. Mol Immunol. 2008; 45:3832-9), and Wolfguy numbering scheme (Bujotzek et al., Proteins 2015; 83(4):681-95). Unless otherwise specified, the numbering scheme used herein for identification of a particular antibody region or domain is the IMGT numbering scheme. Under IMGT unique numbering for the variable and the constant domains of an antibody, conserved amino acids from framework regions always have the same number whatever the immunoglobulin chain type, whatever the domain (variable or constant), and whatever the species they come from. For correspondence with other numbering schemes, see the IMGT Scientific charts as provided on the IMGT website (e.g. https://www.imgt.org/IMGTScientificChart/).

As used herein, the term "*binding*" or "*bind*" refers to the capacity to recognize and contact (or interact with) another entity, herein the antibody is capable recognize and contact (or interact with) an antigen. This binding can entail some complementarity between the antigen binding domain (paratope) of the antibody and its epitope on the antigen, whether this epitope is linear or conformational. According to this definition, an antibody specifically or selectively binds to an antigen when it binds to that antigen via its antigen binding domain more readily than it would to a random, unrelated antigen. In other words, an antibody highly specific to an antigen would typically lack binding, or substantially lack binding, to a random, unrelated antigen. This "*specificity*" or "*selectivity*" in binding can be assessed for example by measuring the relative affinity by which a certain antibody (or antigen-binding fragment thereof) binds to a certain antigen .

The term "*compete with*" with regard to binding can be used herein interchangeably with "*competing*"*,* "*cross-compete with*"*,* "*competitively inhibit*" or "*is a competitive inhibitor of*"*.* An antibody that competes with a reference antibody for a common antigen typically shares the same epitope on that antigen or has an adjacent epitope sufficiently proximal to the epitope of the reference antibody for steric hindrance to occur. The antibody is said to be competing when it inhibits the binding of the reference antibody to a common antigen. This competition is also an indicator of the relative affinity by which the tested antibody binds to the antigen.

By "*affinity*" or "*binding affinity*"*,* it is meant herein the strength of the binding between an antibody and an antigen, such as between the paratope and the epitope. It is well within the skill of the person in the art to assess the affinity of an antibody to an antigen, by methods well-known in the art, such as ELISA (Enzyme-Linked Immunosorbent Assay), BLI (Biolayer Interferometry), RIA (radioimmunoassay), SPR (Surface Plasmon Resonance) (see, e.g., Harlow et al., Using Antibodies, A Laboratory Manual, Cold Spring Harbor Press, ed. 1988;, and/or as described in below Examples section 1.10 with regard to BKV. In a conventional antibody/antigen binding assay, binding affinity is typically represented by the equilibrium dissociated constant (K_{D}), which is a ratio of the rate at which the antibody binds its target antigen (Kₒₙ) and of the rate at which the antibody-antigen complex dissociates (K_{off}). K_{D} and affinity are inversely related: a high affinity interaction is characterized by a low K_{D}, a fast recognizing (high Kₒₙ) and a strong stability of formed complexes (low K_{off}). Antibodies with a higher affinity for a particular antigen would be expected to bind more strongly and stably to said antigen. In the context of the present invention, the K_{D} of antibodies with a high affinity for the antigen is preferably equal or lower than about 150 nM, more preferably equal or lower than about 125 nM, 100 nM, 50 nM, 10 nM, 5 nM, or 1 nM. As an alternative, an apparent estimate of binding affinity can be provided by measuring the half-maximal effective concentration (EC₅₀), which, in a binding assay, is the concentration of antibody at which half (50%) of its target antigen is bound thereto. In the context of the present invention, the EC₅₀ of antibodies with a high affinity for the antigen is preferably equal or lower than about 1.5 µg/ml, more preferably equal or lower than about 1 µg/ml, 0.75 µg/ml, 0.5 µg/ml, 0.1µg/ml or 0.075 µg/ml. Competitive binding assays can also be performed between antibodies. Typically, such an assay involves the use of purified antigen bound to a solid surface or cells bearing either of these, an unlabeled test antibody at varying concentration and a labeled reference antibody at a fixed concentration. Competitive inhibition is then measured by determining the amount of label bound to the solid surface or cells in the presence of the test antibody.

By "*neutralizing*" or "*neutralization*" of an antigen, it is meant herein the capacity to inhibit or reduce the activity of said antigen, which, for an infectious or pathogenic antigen, means the capacity to inhibit or reduce the infectious or pathogenic activity, such as viral replication. This is an important property to assess since not all antibodies that bind to an infectious or pathogenic antigen are neutralizing. It is well within the skill of the person in the art to assess the neutralization activity of an antibody against such antigen, by methods well-known in the art named neutralization assays, such as pseudotype virus assay, microneutralization assay, plaque reduction assay, and/or as described in below Examples section 1.8 with regard to BKV (or JCV). Antibody neutralization can be represented by the IC₅₀ (half-maximal inhibitory concentration), which, in a neutralization assay, is the concentration of antibody at which half (50%) of the antigen activity is inhibited. In the context of the present invention, the IC₅₀ of antibodies with a high neutralization against the antigen is preferably equal or lower than about 75 ng/mL, more preferably equal or lower than about 50 ng/mL, 10 ng/mL, 75 ng/mL, 1 ng/mL, or 0.75 ng/mL. As an alternative, antibody neutralization can be provided by measuring the IC₉₀, which, in a neutralization assay, is the concentration of antibody at which 90% of the antigen activity is inhibited.

The term "*polyreactivity*" or "*polyreactive*" used in reference to an antibody means that said antibody binds to, or reacts with, one or more structurally unrelated antigens. This reactivity is due to low-to-moderate affinity of the antibody against such antigens. Methods commonly known in the art to detect polyreactivity include, without limitation, enzyme-linked immunosorbent assay, immunofluorescence assay, radioimmunoassay, chemiluminescence immunoassay, and immunoblotting. By "*autoreactivity*", "*self-reactivity*", "*autoreactive*" or "*self-reactive*", it is means that an antibody binds to, or reacts with, one or more self-antigens, and as such, may trigger an unwanted immune response against said antigen. Autoreactive antigens may also be referred as autoantibodies.

"*BKV*" or "*BK virus*" (also known as "*human polyomavirus 1*", or "*BK polyomavirus*") and "*JCV*" or "*JC virus*" (also known as "*human polyomavirus 2*"*,* "*JC polyomavirus*" or "*John Cunningham virus*") are related members of the *Polyomaviridae* family, belonging more specifically to the *betapolyomavirus* genus. These polyomaviruses are icosahedral, non-enveloped, viruses measuring approximately 40-45 nM in diameter with a circular double-stranded DNA genome of approximately 5,000 base pairs (Bennett et al., Microbes and Infection 2012; 14(9): 672-683; Johne et al., Arch. Virol. 2011; 156(9): 1627-1634). These polyomaviruses were both originally isolated from immunosuppressed patients in 1971 (Gardner et al., Lancet. 1971; 1 (7712): 1253-7; Padgett et al., Lancet. 1971; 1 (7712): 1257-60 ) and share 75% sequence similarity in their genome. Both of these viruses can be easily identified and differentiated from each other by carrying out serological tests using specific commercial antibodies or a PCR-based genotyping approach. Genetic heterogeneity in the viral protein 1 (VP1) has allowed to further classify BKV isolates into four main subtypes or genotypes (I-IV). BKV subtype I is the most prevalent in the world (80%) followed by subtype IV (15%), subtypes II and III being rather rare. Subgroups within BKV subtypes have also been identified (Morel et al., J Clin Microbiol 2017; 55(4): 1177-1185), such as subgroup Ia, Ib2, Ic, etc. When referring to a plurality of BKV subtypes, it is meant herein at least two BKV subtypes, preferably at least three BKV subtypes.

"*VP1*" or "*viral protein* 1" refers herein to the major BKV or JCV capsid protein that is exposed on the virion surface when the virus enters a host cell, and that forms a pentamer having affinity for the ganglioside receptor on a host cell. It is the binding of VP1 pentamers (i.e. of five VP1 monomers) to ganglioside receptors on the cell surface which initiates internalization through a caveolae-mediated endocytic pathway, followed by trafficking of the virus to the ER and finally to the nucleus where it would replicate. In some assays that may be used in the present invention, VP1 may be provided in the form of VP1 pentamers, pseudovirions, or virus-like particles. A protocol for preparing pseudovirions or VP1 pentamers is provided in below Examples, sections 1.4 or 1.3, respectively. Examples of sequences of a VP1 monomer is provided in Table 1 below for information.

**Table 1. VP1 sequence and naturally-occurring variants thereof**

| **Name** | **Sequence** | **SEQ ID NO:** |
|---|---|---|
| **VP1 BKV subtype I** | MAPTKRKGECPGAAPKKPKEPVQVPKLLIKGGVEVLEVKTGVDAITEVEC FLNPEMGDPDENLRGFSLKLSAENDFSSDSPERKMLPCYSTARIPLPNLN EDLTCGNLLMWEAVTVQTEVIGITSMLNLHAGSQKVHEHGGGKPIQGSN FHFFAVGGEPLEMQGVLMNYRSKYPDGTITPKNPTAQSQVMNTDHKAYL DKNNAYPVECWVPDPSRNENARYFGTFTGGENVPPVLHVTNTATTVLLD EQGVGPLCKADSLYVSAADICGLFTNSSGTQQWRGLARYFKIRLRKRSV KNPYPISFLLSDLINRRTQRVDGQPMYGMESQVEEVRVFDGTERLPGDP DMIRYIDKQGQLQTKML | SEQ ID NO: 40 |
| | Amino acids may be mutated at position 60 (D60N), 68 (L68Q), 72 (A72E), 73 (E73Q), 75 (D75N), and/or 82 (E82D) | - |
| **VP1 BKV subtype II** | MAPTKRKGECPGAAPKKPKEPVQVPKLLIKGGVEVLEVKTGVDAITEVEC FLNPEMGDPDDNLRGYSLKLTAENAFDSDSPDKKMLPCYSTARIPLPNLN EDLTCGNLLMWEAVTVKTEVIGITSMLNLHAGSQKVHENGGGKPVQGSN FHFFAVGGDPLEMQGVLMNYRTKYPQGTITPKNPTAQSQVMNTDHKAYL DKNNAYPVECWIPDPSRNENTRYFGTYTGGENVPPVLHVTNTATTVLLD EQGVGPLCKADSLYVSAADICGLFTNSSGTQQWRGLARYFKIRLRKRSV KNPYPISFLLSDLINRRTQKVDGQPMYGMESQVEEVRVFDGTEQLPGDP DMIRYIDRQGQLQTKMV | SEQ ID NO: 41 |
| **VP1 BKV subtype IV** | MAPTKRKGECPGAAPKKPKEPVQVPKLLIKGGVEVLEVKTGVDAITEVEC FLNPEMGDPDNDLRGYSLRLTAETAFDSDSPDRKMLPCYSTARIPLPNLN EDLTCGNLLMWEAVTVKTEVIGITSMLNLHAGSQKVHENGGGKPIQGSN FHFFAVGGDPLEMQGVLMNYRTKYPEGTVTPKNPTAQSQVMNTDHKAY LDKNNAYPVECWIPDPSRNENTRYFGTYTGGENVPPVLHVTNTATTVLL DEQGVGPLCKADSLYVSAADICGLFTNSSGTQQWRGLPRYFKIRLRKRS VKNPYPISFLLSDLINRRTQRVDGQPMYGMESQVEEVRVFDGTEQLPGD PDMIRYIDRQGQLQTKMV | SEQ ID NO: 41 |
| | Amino acids may be mutated at position 77(D77Q) | |
| **VP1 JCV** | MAPTKRKGERKDPVQVPKLLIRGGVEVLEVKTGVDSITEVECFLTPEMGD PDEHLRGFSKSISISDTFESDSPNRDMLPCYSVARIPLPNLNEDLTCGNIL MWEAVTLKTEVIGVTSLMNVHSNGQATHDNGAGKPVQGTSFHFFSVGG EALELQGVLFNYRTKYPDGTIFPKNATVQSQVMNTEHKAYLDKNKAYPVE CWVPDPTRNENTRYFGTLTGGENVPPVLHITNTATTVLLDEFGVGPLCK GDNLYLSAVDVCGMFTNRSGSQQWRGLSRYFKVQLRKRRVKNPYPISF LLTDLINRRTPRVDGQPMYGMDAQVEEVRVFEGTEELPGDPDMMRYVD KYGQLQTKML | SEQ ID NO: 43 |

The term "*amino acid*" or "*amino acid residue*" refers to a molecule containing both an amino group and a carboxyl group, with no peptide bond within the molecule. In other words, an amino acid is a molecule that has a carboxylic acid functionality and an amine nitrogen having at least one free hydrogen, preferably in alpha position thereto, but no amide bond in the molecule structure. This term encompasses not only naturally-occurring amino acids but also, synthetic, and unnatural amino acids, as well as amino acid analogs and amino acid mimetics that function in a manner similar to naturally occurring amino acids. Naturally occurring amino acids are those encoded by the genetic code (e.g. Alanine (Ala, A), Arginine (Arg, R), Asparagine (Asn, N), Aspartic acid (Asp, D), Cysteine (Cys, C), Glutamine (Gln, Q), Glutamic acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y) and Valine (Val, V)) in D or L form, preferably in L form, as well as those amino acids that are later modified, e.g., hydroxyproline, g-carboxyglutamate, and O-phosphoserine. Amino acid analogs refer to compounds that have the same basic chemical structure as a naturally occurring amino acid, i.e., an a-carbon that is bound to a hydrogen, a carboxyl group, an amino group, and a side chain (R group), e.g., homoserine, norleucine, methionine sulfoxide, methionine methyl sulfonium. Such analogs have modified side chains (see e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. In the context of the present invention, amino acids are herein preferably those that are naturally-occurring.

Amino acids are the building blocks of proteins, such as of antibodies or VP 1 as disclosed herein. The term "*polypeptide*" or "*protein*" refers herein to a precise succession of amino acid residues, also referred as amino acid sequence. As such, these terms include polypeptides of any size, preferably of at least 200, 300, 400, 500, 600, 700 amino acids or more, and/or polypeptides that have undergone post-translational modifications. In the context of the present invention, human monoclonal antibodies disclosed herein, or the VP1 protein they target, may be defined by sequence identity to a reference sequence, and/or contain amino acid mutation(s) which retain, or substantially retain, their biological activity. Herein, it will be understood that the biological activity of the human monoclonal antibodies according to the invention includes at least the binding and/or neutralizing of BK virus, while the biological activity of VP1 includes at least the binding of VP1 to ganglioside receptors on the host cell surface.

"*Sequence identity*" between amino acid sequences can be determined by comparing a position in each of the sequences which may be aligned for the purposes of comparison. When a position in the compared sequences is occupied by the same amino acid, then the sequences are identical at that position. A degree of identity between amino acid sequences is a function of the number of identical amino acid sequences that are shared between these sequences. To determine the "*percentage of sequence identity*" between two amino acid sequences, the sequences can be aligned for optimal comparison. For example, gaps can be introduced in the sequence of a first amino acid sequence for optimal alignment with the second amino acid sequence. The amino acid residues at corresponding amino acid positions can then compared. When a position in the first sequence is occupied by the same amino acid residue as the corresponding position in the second sequence, the molecules are identical at that position. The percentage (%) of identity between the two sequences is a function of the number of identical positions shared by the sequences. Hence, the percentage of identity can be calculated by multiplying the number of identical positions by 100 and dividing by the length of the aligned region (overlapping positions), including gaps (only internal gaps, not the gaps at the sequence ends). In this comparison, the sequences can be of the same length, or may be of different lengths. Identity scoring only counts perfect matches, and does not consider the degree of similarity of amino acids to one another. The percentage of identity can be calculated over the entire length of the sequence of reference, or over the entire relevant section thereof (if, for example, the percentage of sequence identity does not apply to one or more sections of the sequence of reference). Optimal alignment of sequences may be conducted by a global homology alignment algorithm, such as by the one described by Needleman and Wunsch (Journal of Molecular Biology 1970, 48(3): 443-53), by a local homology alignment algorithm such as the one described by Smith-Waterman et al. (Journal of Molecular Biology 1981; 147 (1): 195-197), by computerized implementations of either of these algorithms (for a global alignment, see e.g. the EMBOSS Needle program available online from EMBL-EBI; for a local alignment, see e.g. the BLAST program available online from NCBI), or by mere visual inspection. A global homology alignment may be preferred if the sequences to be aligned have the same or similar length. Alternatively, one may favor a local alignment if the sequences to be aligned are substantially different in their length.

The term "*modification*" or "*modified*" with regard to a polypeptide is intended to refer to an alteration or change in or to the polypeptide. Such modification may be desired, for example, to optimize the properties of the polypeptide or eliminate undesired properties of the polypeptide. Examples of such modification include those to remove glycosylation sites, GPI anchors, and/or solvent exposed lysines, to name a few. These modifications may be achieved by engineering a mutation of the relevant amino acid in the polypeptide, or by chemical or enzymatic treatment of the relevant amino acid residue such as acetylation, amidation, hydroxylation, methylation, phosphorylation, derivatization by known protecting/blocking groups, and/or attachment of a carbohydrate or lipid moieties, cofactors and the like.

The term "*mutated*" or "*mutation*" refers to an alteration or change of an amino acid in a reference polypeptide, such as by amino acid substitution, insertion and/or deletion. A "*substitution*" refers to the replacement of an amino acid at a particular sequence position in a reference polypeptide by another amino acid; an "*insertion*" to the addition of an amino acid at a particular sequence position in a reference polypeptide; and a "*deletion*" to the removal an amino acid at a particular sequence position in a reference polypeptide. Silent mutations, conservative mutations, or minor deletions are herein particularly preferred.

"*Conservative substitutions*" are a particularly preferred type of conservative mutations or modifications. This term refers to an amino acid substitution which does not alter the overall conformation and function of a polypeptide of reference, including, preferably the replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

Examples of conservative substitutions are set out in Table 2 below.

**Table 2. Conservative substitutions I**

| **Side chain characteristic** | **Amino Acid** |
|---|---|
| Non-polar | G, A, P, I, L, V |
| Polar uncharged | C, S, T, M, N, Q |
| Polar-charged | D, E, K, R |
| Aromatic | H, F, W, Y |
| Other | N, Q, D, E |

Alternatively, conservative amino acids can be grouped as described in Lehninger, 1975, as set out in Table 3 below.

**Table 3. Conservative substitutions II**

| **Side chain characteristic** | **Amino Acid** |
|---|---|
| *Non-polar (hydrophobic)* | |
| Aliphatic | A, L, I, V, P |
| Aromatic | F, W |
| Sulfur-containing | M |
| Borderline | G |

| *Polar uncharged* | |
|---|---|
| Hydroxyl | S, T, Y |
| Amides | N, Q |
| Sulfhydryl | C |
| Borderline | G |

| *Polar-charged* | |
|---|---|
| Positively charged (basic) | K, R, H |
| Negatively charged (acidic) | D, E |

As a further alternative, exemplary conservative substitutions are set out in Table 4 below.

**Table 4. Conservative substitutions III**

| **Original amino acid residue** | **Amino acid substitution** |
|---|---|
| Ala (A) | Val (V), Leu (L), Ile (I) |
| Arg (R) | Lys (K), Gln (Q), Asn (N) |
| Asn (N) | Gln (Q), His (H), Lys(K), Arg (R) |
| Asp (D) | Glu (E) |
| Cys (C) | Ser (S) |
| Gln (Q) | Asn (N) |
| Glu (E) | Asp (D) |
| His (H) | Asn (N), Gln (Q), Lys (K), Arg (R) |
| Ile (I) | Leu (L), Val (V), Met (M), Ala (A), Phe (F) |
| Leu (L) | Ile (I), Val (V), Met (M), Ala (A), Phe (F) |
| Lys (K) | Arg (R), Gln (Q), Asn (N) |
| Met (M) | Leu (L), Phe (F), Ile (I) |
| Phe (F) | Leu (L), Val (V), Ile (I), Ala (A) |
| Pro (P) | Gly (G) |
| Ser (S) | Thr (S) |
| Thr (T) | Ser (S) |
| Trp (W) | Tyr (T) |
| Tyr (Y) | Trp (W), Phe (F), Thr (T), Ser (S) |
| Val (V) | Ile (I), Leu (L), Met (M), Phe (F), Ala (A) |

The term "*isolated*" means herein that a material has been removed or separated from its natural environment or otherwise subjected to human manipulation. The isolated material may be substantially or essentially free from components that normal accompany it in its natural states (e.g. having a degree of purity greater than 90%, 95% or 99%). This term may apply to any biological material disclosed herein, such as the antibody according to the invention. For example, the antibody according to the invention, can be substantially free of cellular material or other contaminating proteins from the cell or tissue source from which it was derived, or substantially free of chemical precursors or other chemicals when chemically synthesized.

Generally speaking, the term "*treatment*" or "*treating*" means obtaining a desired physiological or pharmacological effect depending on the degree of severity of the symptom or disorder of interest, or risks thereof, i.e. herein, depending on the degree of severity of the BKV or JCV infection or associated disorder thereof, or risks of developing such infection or associated disorder. The effect may thus be prophylactic in terms of a partial or complete prevention of the symptom or disorder and/or may be therapeutic in terms of a partial or complete cure of the symptom or disorder. The term "*prophylactic*" characterizes the capacity to avoid, or minimize the onset or development of a symptom or disorder before its onset (for example, after exposure to BKV or JCV, but before the onset of associated symptom). The term "*therapeutic*" refers to the capacity to inhibit the symptom or disorder (i.e. arresting the development thereof), and/or to relieve said symptom or disorder (i.e. regression leading to an improvement).

"*BKV or JCV infection*" refers herein either to the passive or active entry of the BKV or JCV virus into a host cell and subsequent increase in viral load (i.e. viral replication) within the host. This term encompasses a primary BKV or JCV infection, as well as a secondary BKV or JCV infection (i.e. after reactivation of the virus). Such infection may be non-pathogenic, or subclinical in its manifestation, at least initially.

"*BKV of JCV-associated disorder*" refers herein to a disorder in a host that has been infected by BKV or JCV. Typical examples of BKV- of JCV-associated disorders include, without limitation, graft dysfunction or rejection (such as kidney graft), BKV-related leukodystrophy, nephropathy, BK virus-associated nephritis (BKVAN), hemorrhagic cystitis, Progressive Multifocal Leukoencephalopathy (PML), granule cell neuronopathy (GCN), interstitial kidney disease, ureteral stenosis, urothelial carcinoma, vasculitis, colitis, retinitis, meningitis, pneumonitis, liver disease, and immune reconstitution inflammatory syndrome (IRIS).

The term "*about*" means that the value of reference can vary within a certain range depending on the margin of error allowed, which may be easily determined by one skilled in the art. Preferably, this margin of error is of 10%, more preferably of 5%, even more preferably of 1%.

### Antibodies

The present invention relates to a set of monoclonal antibodies, preferably human monoclonal antibodies, and antigen-binding fragments or chains thereof, which bind and neutralize a plurality of BK virus (BKV) subtypes including at least subtypes I, II and IV. They bind more particularly to the VP1 protein, and efficiently neutralize viral infection from BK virus. The antibodies or antigen-binding fragments according to the invention can further bind and neutralize JC virus.

The antibodies disclosed herein are defined by their complementary-determining regions (CDRs), which are key determinants in antigen binding, the CDRs being determined according to the IMGT numbering scheme. The antibodies can also be defined by their heavy chain variable domain (VH) and light chain variable domain (VL), also determined according to the IMGT numbering scheme. Consensus sequences of CDRs and variable domains are provided herein. Specific sequences of CDRs and variable domains of the antibodies are also disclosed in Tables 5 and 6 below. It will be appreciated that mutants of these CDRs and variable domains may also be used herein, which retain or substantially retain the capacity for binding to VP1 and accordingly retain or substantially retain the capacity for neutralizing BKV/ JCV.

The present invention further encompasses other neutralizing antibodies that (1) compete for binding to naturally-occurring VP1 with the above-described antibodies; (2) bind to the same or similar epitope on naturally-occurring VP 1 as those antibodies, and/or (3) lose binding or have a reduced binding when the VP1 epitope is mutated.

### CDRs regions

In a first aspect, a human monoclonal antibody or antigen-binding fragment thereof according to the invention binds and neutralizes a plurality of BK virus subtypes including at least subtypes I, II and IV, and has a combination of a heavy chain variable domain (VH) and a light chain variable domain (VL) with the six following complementary-determining regions (CDRs):
- a VH-CDR1 comprising, or consisting of, amino acid sequence GFX₁X₂RX₃YX₄ (SEQ ID NO: 1), wherein X₁ is S, T or N; X₂ is F, I or L I; X₃ is S or G; and X₄ is A or G;
- a VH-CDR2 comprising, or consisting of, amino acid sequence LX₅SX₆GX₇X₈X₉, wherein X₅ is N, T, S or R; X₆ is A, S or N; X₇ is V, A or T; X₈ is S or T; X₉ is T or A;
- a VH-CDR3 comprising, or consisting of, amino acid sequence AX₁₀DRX₁₁X₁₂ X₁₃WLGSEPX₁₄DP (SEQ ID NO: 2), wherein X₁₀ is K or R; X₁₁ is G, D or N,; X₁₂ is V or A; X₁₃ is E or Q; X₁₄ is I, F or L;
- a VL-CDR1 comprising, or consisting of, amino acid sequence QX₁₅X₁₆X₁₇X₁₈X₁₉Y, wherein X₁₅ is R or S; X₁₆ is V or L; X₁₇ is S or G; X₁₈ is S or N; X₁₉ is N or S;
- a VL-CDR2 comprising, or consisting of, amino acid sequence X₂₀AS, wherein X₂₀ is R, G or D; and
- a VL-CDR3 comprising, or consisting of, amino acid sequence QQYGSX₂₁PRS (SEQ ID NO: 3), wherein X₂₁ is S or P.

In a preferred embodiment, the six complementary-determining regions (CDRs) of the human monoclonal antibody or antigen-binding fragment thereof according to the invention are:
- a VH-CDR1 comprising, or consisting of, amino acid sequence GFX₁X₂RX₃YX₄ (SEQ ID NO: 1), wherein X₁ is S; X₂ F or I; X₃ is S or G; and X4 is A;
- a VH-CDR2 comprising, or consisting of, amino acid sequence LX₅SX₆GX₇X₈X₉, wherein X₅ is N or T; X₆ is A or N; X₇ V or A; X₈ is S or T; X₉ is T;
- a VH-CDR3 comprising, or consisting of, amino acid sequence AX₁₀DRX₁₁X₁₂ X₁₃WLGSEPX₁₄DP (SEQ ID NO: 2), wherein X₁₀ is K; X₁₁ is G or D; X₁₂ is V or A; X₁₃ is E; X₁₄ is I or F;
- a VL-CDR1 comprising, or consisting of, amino acid sequence QX₁₅X₁₆X₁₇X₁₈X₁₉Y, wherein X₁₅ is R ; X₁₆ is V; X₁₇ is S; X₁₈ is S; X₁₉ is N or S;
- a VL-CDR2 comprising, or consisting of, amino acid sequence X₂₀AS, wherein X₂₀ is R or G; and
- a VL-CDR3 comprising, or consisting of, amino acid sequence QQYGSX₂₁PRS (SEQ ID NO: 3), wherein X₂₁ is S.

Particularly preferred examples of complementary-determining regions (CDRs) of the antibody or antigen-binding fragment thereof according to the invention are:
- a VH-CDR1 comprising, or consisting of, amino acid sequence SEQ ID NO: 6, 13, 19, 25, 28 or 34;
- a VH-CDR2 comprising, or consisting of, amino acid sequence SEQ ID NO: 7, 14, 20, 29 or 35;
- a VH-CDR3 comprising, or consisting of, amino acid sequence SEQ ID NO: 8, 15, 30 or 36;
- a VL-CDR1 comprising, or consisting of, amino acid sequence SEQ ID NO: 9, 16, 21, 31 or 37;
- a VL-CDR2 comprising, or consisting of, amino acid sequence RAS, GAS or DAS; and
- a VL-CDR3 comprising, or consisting of, amino acid sequence SEQ ID NO: 10 or 22.

Even more preferred examples of complementary-determining regions (CDRs) of the antibody or antigen-binding fragment thereof according to the invention are:
- a VH-CDR1 comprising, or consisting of, amino acid sequence SEQ ID NO: 6 or 13 ;
- a VH-CDR2 comprising, or consisting of, amino acid sequence SEQ ID NO: 7 or 14;
- a VH-CDR3 comprising, or consisting of, amino acid sequence SEQ ID NO: 8 or 15;
- a VL-CDR1 comprising, or consisting of, amino acid sequence SEQ ID NO: 9 or 16;
- a VL-CDR2 comprising, or consisting of, amino acid sequence RAS or GAS; and
- a VL-CDR3 comprising, or consisting of, amino acid sequence SEQ ID NO: 10.

More preferably, the six complementary determining regions (CDRs) of the antibody or antigen-binding fragment according to the invention are selected from either of the following combinations, as shown in Table 5:
(a) VH-CDR1, VH-CDR2 and VH-CDR3 comprising, or consisting of, amino acid sequence SEQ ID NO: 6, 7 and 8, respectively; and VL-CDR1, VL-CDR2 and VL-CDR3 comprising, or consisting of, amino acid sequence SEQ ID NO: 9, RAS and SEQ ID NO: 10, respectively; or
(b) VH-CDR1, VH-CDR2 and VH-CDR3 comprising, or consisting of, amino acid sequence SEQ ID NO: 13, 14 and 15, respectively; and VL-CDR1, VL-CDR2 and VL-CDR3 comprising, or consisting of, amino acid sequence SEQ ID NO: 16, GAS and SEQ ID NO: 10, respectively; or
(c) VH-CDR1, VH-CDR2 and VH-CDR3 comprising, or consisting of, amino acid sequence SEQ ID NO: 19, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(d) VH-CDR1, VH-CDR2 and VH-CDR3 comprising, or consisting of, amino acid sequence SEQ ID NO: 25, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(e) VH-CDR1, VH-CDR2 and VH-CDR3 comprising, or consisting of, amino acid sequence SEQ ID NO: 28, 29 and 30, respectively; and VL-CDR1, VL-CDR2 and VL-CDR3 comprising, or consisting of, amino acid sequence SEQ ID NO: 31, GAS and SEQ ID NO: 10, respectively; or
(f) VH-CDR1, VH-CDR2 and VH-CDR3 comprising, or consisting of, amino acid sequence SEQ ID NO: 34, 35 and 36, respectively; and VL-CDR1, VL-CDR2 and VL-CDR3 comprising, or consisting of, amino acid sequence SEQ ID NO: 37, GAS and SEQ ID NO: 10, respectively.

Even more preferably, the six complementary determining regions (CDRs) of the antibody or antigen-binding fragment according to the invention are selected from either of the following combinations:
(a) VH-CDR1, VH-CDR2 and VH-CDR3 comprising, or consisting of, amino acid sequence SEQ ID NO: 6, 7 and 8, respectively; and VL-CDR1, VL-CDR2 and VL-CDR3 comprising, or consisting of, amino acid sequence SEQ ID NO: 9, RAS and SEQ ID NO: 10, respectively; or
(b) VH-CDR1, VH-CDR2 and VH-CDR3 comprising, or consisting of, amino acid sequence SEQ ID NO: 13, 14 and 15, respectively; and VL-CDR1, VL-CDR2 and VL-CDR3 comprising, or consisting of, amino acid sequence SEQ ID NO: 16, GAS and SEQ ID NO: 10, respectively.

It will be appreciated that mutants of the above-described CDRs may also be used herein, which retain or substantially retain the capacity for binding to VP1. For example, 1, 2 or 3 amino acids may be mutated in one or more of those CDRs, such as by substitution, deletion and/or insertion, preferably by substitution, more preferably by conservative substitution.

### Variable domains VH and VL

As explained above, the VH and VL variable domains are subdivided into regions of hypervariability, termed complementarity determining regions (CDR), which are interspersed with regions that are more conserved, termed framework regions (FR).

Because CDRs are responsible for most antibody-antigen interactions, the above-described CDRs may be grafted onto any suitable antibody framework regions, without impacting, or without substantially impacting, the biological activity of the antibody or antigen-binding fragment thereof according to the invention.

In the context of the present invention, it will be understood that human antibody frameworks are herein preferred. Human framework sequences can be easily obtained from e.g. publicly available databases such as http://www2.mrc-imb.cam.ac.uk/vbase/list2.php, or https://vdjbase.org/. If need be, those framework regions may also be mutated. For example, 1, 2, 3, 4, 5, 6, 7, 8, 9, or 10 amino acids may be mutated in one or more framework regions such as by substitution, deletion and/or insertion, preferably by substitution, more preferably by conservative substitution. Protocols for selecting amino acid residues in framework regions which can mutated are well-known in the art.

In a preferred embodiment, the variable domains of the antibody or antigen-binding fragment thereof according to the invention are as follows:
- the heavy chain variable domain (VH) comprises, or consists of, an amino acid sequence having at least 80%, 85%, 90%, 91%, 92%, 93%, 94%, 95%, 96%, 97%, 98% or 99% sequence identity to EVQLLESGGX₂₂LVQPGGSLRLSCX₂₃ASGFX₁X₂RX₃Y X₄MNWVRQAPGX₂₄GLEWVSSLX₅SX₆GX₇X₈X₉YYAX₂₅SVKGRFTISRDNX₂₆KN TX₂₇X₂₈LQMX₂₉SLRGEDTAVYYCAX₁₀DRX₁₁X₁₂X₁₃WLGSEPX₁₄DPWGQGTX₃₀V TVSS (SEQ ID NO: 4), wherein X₁ to X₁₄ are as described above; X₂₂ is G or A; X₂₃ is A or V; X₂₄ is K or R; X₂₅ is E or D; X₂₆ is S or F; X₂₇ is L or M; X₂₈ is Y or F; X₂₉ is N, H or D; and X₃₀ is L or M; and
- the light chain variable domain (VL) comprises, or consists of, an amino acid sequence having at least 80%, 85%, 90%, 91%, 92%, 93%, 94%, 95%, 96%, 97%, 98% or 99% sequence identity to EIVLTQSPGTLSLSPGERAT LSCRASQX₁₅X₁₆X₁₇X₁₈ X₁₉ YLAWYQHKX₃₁GX₃₂APRLLIYX₂₀ASX₃₃RAX₃₄GIPDRFSGSX₃₅ SGTDTLTISRLEP EDFAVYYCQQYGSX₂₁PRSFGQGTKLEIK (SEQ ID NO: 5), wherein X₁₅ to X₂₁ are as described above; X₃₁ is P or F; X₃₂ is Q or L; X₃₃ is S or R; X₃₄ is T or I; and X₃₅ is G or E. The CDRs of each variable domain are as described above.

A total of 1 to 10 amino acids may be optionally mutated in the heavy chain variable domain (VH) (SEQ ID NO: 4) and/or in the light chain variable domain (VL) (SEQ ID NO: 5), such as by substitution, deletion and/or insertion, preferably by substitution, more preferably by conservative substitution. Said mutations, if any, are preferably introduced in regions outside the CDRs, or in other words, the CDRs are preferably not mutated. To put it another way, said mutations, if any, are preferably introduced in the framework regions. Optionally, amino acid X₄₉ in the light chain variable domain (VL) (SEQ ID NO: 5) is not mutated.

More precisely, the variable domains of the antibody or antigen-binding fragment thereof according to the invention are as follows:
- the heavy chain variable domain (VH) comprises, or consists of, an amino acid sequence having at least 80%, 85%, 90%, 91%, 92%, 93%, 94%, 95%, 96%, 97%, 98% or 99% sequence identity to EVQLLESGGX₂₂LVQPGGSLRLSCX₂₃ASGFX₁X₂RX₃Y X₄MNWVRQAPGX₂₄GLEWVSSLX₅SX₆GX₇X₈X₉YYAX₂₅SVKGRFTISRDNX₂₆KN TX₂₇X₂₈LQMX₂₉SLRGEDTAVYYCAX₁₀DRX₁₁X₁₂X₁₃WLGSEPX₁₄DPWGQGTX₃₀V TVSS (SEQ ID NO: 4), wherein X₁ is S; X₂ is F or I; X₃ is S or G; X₄ is A; X₅ is N or T; X₆ is A or N; X₇ is V or A; X₈ is S or T; X₉ is T; X₁₀ is K; X₁₁ is G or D; X₁₂ is V or A; X₁₃ is E; X₁₄ is I or F; X₂₂ is G; X₂₃ is A or V; X₂₄ is K; X₂₅ is E or D; X₂₆ is S; X₂₇ is L; X₂₈ is Y or F; X₂₉ is N or H; and X₃₀ is L; and
- the light chain variable domain (VL) comprises, or consists of, an amino acid sequence having at least 80%, 85%, 90%, 91%, 92%, 93%, 94%, 95%, 96%, 97%, 98% or 99% sequence identity to EIVLTQSPGTLSLSPGERAT LSCRASQX₁₅X₁₆X₁₇X₁₈X₁₉ YLAWYQHKX₃₁GX₃₂APRLLIYX₂₀ASX₃₃RAX₃₄GIPDRFSGSX₃₅SGTDTLTISRLEP EDFAVYYCQQYGSX₂₁PRSFGQGTKLEIK (SEQ ID NO: 5), wherein X₁₅ is R; X₁₆ is V; X₁₇ is S; X₁₈ is S; X₁₉ is S; X₂₀ is R or G; X₂₁ is S; X₃₁ is P or F; X₃₂ is Q; X₃₃ is S or R; X₃₄ is T; and X₃₅ is G.

The CDRs of each variable domain are as described above.

A total of 1 to 10 amino acids may be optionally mutated in the heavy chain variable domain (VH) (SEQ ID NO: 4) and/or in the light chain variable domain (VL) (SEQ ID NO: 5), such as by substitution, deletion and/or insertion, preferably by substitution, more preferably by conservative substitution. Said mutations, if any, are preferably introduced in regions outside the CDRs, or in other words, the CDRs are preferably not mutated. To put it another way, said mutations, if any, are preferably introduced in the framework regions. Optionally, amino acid X₄₉ in the light chain variable domain (VL) (SEQ ID NO: 5) is not mutated.

Particularly preferred examples of variable domains of the antibody or antigen-binding fragment thereof according to the invention are:
- a VH comprising, or consisting of, an amino acid sequence having at least 80%, 85%, 90%, 91%, 92%, 93%, 94%, 95%, 96%, 97%, 98% or 99% sequence identity to any one of SEQ ID NO: 11, 17, 23, 26, 32 or 38; and
- a VL comprising, or consisting of, an amino acid sequence having at least 80%, 85%, 90%, 91%, 92%, 93%, 94%, 95%, 96%, 97%, 98% or 99% sequence identity to any one of SEQ ID NO: 12, 18, 24, 27, 33 or 39.

The CDRs of each of those variable domains are as described above.

A total of 1 to 10 amino acids may be optionally mutated in the heavy chain variable domain (VH) (SEQ ID NO: 11, 17, 23, 26, 32 or 38) and/or in the light chain variable domain (VL) (SEQ ID NO: 12, 18, 24, 27, 33 or 39), such as by substitution, deletion and/or insertion, preferably by substitution, more preferably by conservative substitution. Said mutations, if any, are preferably introduced in regions outside the CDRs, or in other words, the CDRs are preferably not mutated. To put it another way, said mutations, if any, are preferably introduced in the framework regions. Accordingly, the amino acid sequence has at least 80%, 85%, 90%, 91%, 92%, 93%, 94%, 95%, 96%, 97%, 98% or 99% sequence identity to the recited sequence, with the proviso that the CDRs are not mutated, the CDRs being preferably as shown in Table 5 or underlined in Table 6. Optionally, amino acid R54 in the VL domain of SEQ ID NO: 12, or amino acid S54 in the VL domain of SEQ ID NO: 18, is not mutated.

For illustrative purpose, one preferred example (referred as example (i)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 11 and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 12;
with the proviso that the CDRs are:
- VH-CDR1, VH-CDR2 and VH-CDR3 comprising, or consisting of, amino acid sequence SEQ ID NO: 6, 7 and 8, respectively; and VL-CDR1, VL-CDR2 and VL-CDR3 comprising, or consisting of, amino acid sequence SEQ ID NO: 9, RAS and SEQ ID NO: 10, respectively.

Still, more preferred variable domains according to example (i) are those of antibody E3P7, as shown in Table 6:
- a VH comprising, or consisting of, amino acid sequence SEQ ID NO: 11; and
- a VL comprising, or consisting of, amino acid sequence SEQ ID NO: 12.

For illustrative purpose, another preferred example (referred as example (ii)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 17; and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 18;
with the proviso that the CDRs are:
- VH-CDR1, VH-CDR2 and VH-CDR3 comprising, or consisting of, amino acid sequence SEQ ID NO: 13, 14 and 15, respectively; and VL-CDR1, VL-CDR2 and VL-CDR3 comprising, or consisting of, amino acid sequence SEQ ID NO: 16, GAS and SEQ ID NO: 10, respectively.

Still, more preferred variable domains according to example (ii) are those of antibody D10P4, as shown in Table 6:
- a VH comprising, or consisting of, amino acid sequence SEQ ID NO: 17; and
- a VL comprising, or consisting of, amino acid sequence SEQ ID NO: 18.

For illustrative purpose, another preferred example (referred as example (iii)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 23; and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 24;
with the proviso that the CDRs are:
- VH-CDR1, VH-CDR2 and VH-CDR3 comprising, or consisting of, amino acid sequence SEQ ID NO: 19, 20 and 15, respectively; and VL-CDR1, VL-CDR2 and VL-CDR3 comprising, or consisting of, amino acid sequence SEQ ID NO: 21, DAS and SEQ ID NO: 22, respectively.

Still, more preferred variable domains example (iii) are those of antibody D6P7, as shown in Table 6:
- a VH comprising, or consisting of, amino acid sequence SEQ ID NO: 23; and
- a VL comprising, or consisting of, amino acid sequence SEQ ID NO: 24.

For illustrative purpose, another preferred example (referred as example (iv)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 26; and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 27;
with the proviso that the CDRs are:
- VH-CDR1, VH-CDR2 and VH-CDR3 comprising, or consisting of, amino acid sequence SEQ ID NO: 25, 20 and 15, respectively; and VL-CDR1, VL-CDR2 and VL-CDR3 comprising, or consisting of, amino acid sequence SEQ ID NO: 21, DAS and SEQ ID NO: 22, respectively.

Still, more preferred variable domains according to example (iv) are those of antibody E4P7, as shown in Table 6:
- a VH comprising, or consisting of, amino acid sequence SEQ ID NO: 26; and
- a VL comprising, or consisting of, amino acid sequence SEQ ID NO: 27.

For illustrative purpose, another preferred example (referred as example (v)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 32; and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 33;
with the proviso that the CDRs are:
- VH-CDR1, VH-CDR2 and VH-CDR3 comprising, or consisting of, amino acid sequence SEQ ID NO: 28, 29 and 30, respectively; and VL-CDR1, VL-CDR2 and VL-CDR3 comprising, or consisting of, amino acid sequence SEQ ID NO: 31, GAS and SEQ ID NO: 10, respectively.

Still, more preferred variable domains according to example (v) are those of antibody G5P4, as shown in Table 6:
- a VH comprising, or consisting of, amino acid sequence SEQ ID NO: 32; and
- a VL comprising, or consisting of, amino acid sequence SEQ ID NO: 33.

For illustrative purpose, another preferred example (referred as example (vi)) of variable domains of the antibody or antigen-binding fragment thereof according to the invention, in which the CDRs are not mutated, are:
- a VH comprising, or consisting of, an amino acid sequence having at least 80%, 85%, 90%, 91%, 92%, 93%, 94%, 95%, 96%, 97%, 98% or 99% sequence identity to SEQ ID NO: 38; and
- a VL comprising, or consisting of, an amino acid sequence having at least 80%, 85%, 90%, 91%, 92%, 93%, 94%, 95%, 96%, 97%, 98% or 99% sequence identity to SEQ ID NO: 39;
with the proviso that the CDRs are:
- VH-CDR1, VH-CDR2 and VH-CDR3 comprising, or consisting of, amino acid sequence SEQ ID NO: 34, 35 and 36, respectively; and VL-CDR1, VL-CDR2 and VL-CDR3 comprising, or consisting of, amino acid sequence SEQ ID NO: 37, GAS and SEQ ID NO: 10, respectively.

Still, more preferred variable domains according to example (vi) are those of antibody B8P4, as shown in Table 6:
- a VH comprising, or consisting of, amino acid sequence SEQ ID NO: 38; and
- a VL comprising, or consisting of, amino acid sequence SEQ ID NO: 39.

The VH and VL variable domains as shown in above examples (i) and (ii) are herein the more preferred, especially those of example (i).

Indeed, the human monoclonal antibodies E3P7 and D10P4, which comprise these VH and VL domains, exhibit the most potent neutralizing activity towards BKV subtypes I, II and IV, with a high binding affinity to their VP1. These antibodies are also capable of binding and neutralizing JCV, as well as particular naturally-occurring variants of BKV subtypes, such as those comprising VP1 amino acid mutation D60N, L68Q, A72E, E73Q, D75N, and/or E82D (in SEQ ID NO: 40), and/or VP1 amino acid mutation D77Q (in SEQ ID NO: 40).

In a preferred embodiment, the antibody or antigen-binding fragment thereof according to the invention is not an autoreactive and/or polyreactive antibody.

**Table 5. CDRs of the antibodies, or antigen-binding fragments, according to the invention**

| | **VH-CDR1** | | **VH-CDR2** | | **VH-CDR3** | | **VL-CDR1** | | **VL-CDR2** | **VL-CDR3** | |
|---|---|---|---|---|---|---|---|---|---|---|---|
| **Name** | **Sequence** | **SEQ ID NO :** | **Sequence** | **SEQ ID NO :** | **Sequence** | **SEQ ID NO :** | **Sequence** | **SEQ ID NO :** | **Sequence** | **Sequence** | **SEQ ID NO :** |
| **E3P7** | GFSFRSYA | 6 | LNSAGVST | 7 | AKDRGVEWLGSEPIDP | 8 | QRVSSNY | 9 | RAS | QQYGSSPRS | 10 |
| **D10P4** | GFSIRGYA | 13 | LTSNGATT | 14 | AKDRDAEWLGSEPFDP | 15 | QRVSSSY | 16 | GAS | QQYGSSPRS | 10 |
| **D6P7** | GFNFRSYG | 19 | LRSSGVTT | 20 | AKDRDAEWLGSEPFDP | 15 | QSLSNSY | 21 | DAS | QQYGSPPRS | 22 |
| **E4P7** | GFSFRSYG | 25 | LRSSGVTT | 20 | AKDRDAEWLGSEPFDP | 15 | QSLSNSY | 21 | DAS | QQYGSPPRS | 22 |
| **G5P4** | GFSLRGYA | 28 | LTSNGATA | 29 | ARDRDAEWLGSEPFDP | 30 | QSVSSSY | 31 | GAS | QQYGSSPRS | 10 |
| **B8P4** | GFTFRSYA | 34 | LSSSGTTT | 35 | AKDRNAQWLGSEPLDP | 36 | QRVGSNY | 37 | GAS | QQYGSSPRS | 10 |

**Table 6.Variable domain of the antibodies, or antigen-binding fragments, according to the invention(the sequences of the CDRs are underlined**

| | **VH** | | VL | |
|---|---|---|---|---|
| **Name** | **Sequence** | **SEQ ID NO :** | **Sequence** | **SEQ ID NO:** |
| | (FR1-CDR1-FR2-CDR2-FR3-CDR3-FR4) | | (FR1-CDR1-FR2-CDR2-FR3-CDR3-FR4) | |
| **E3P7** | EVQLLESGGGLVQPGGSLRLSCAASGFSFRSYAMNWVRQAPGKG LEWVSSLNSAGVSTYYAESVKGRFTISRDNSKNTLYLQMNSLRGE DTAVYYCAKDRGVEWLGSEPIDPWGQGTLVTVSS | 11 | EIVLTQSPGTLSLSPGERATLSCRASQRVSSNYLAWYQHKPGQAP RLLIYRASSRATGIPDRFSGSGSGTDFTLTISRLEPEDFAVYYCQQY GSSPRSFGQGTKLEIK | 12 |
| **D10P4** | EVQLLESGGGLVQPGGSLRLSCVASGFSIRGYAMNWVRQAPGKG LEWVSSLTSNGATTYYADSVKGRFTISRDNSKNTLFLQMHSLRGED TAVYYCAKDRDAEWLGSEPFDPWGQGTLVTVSS | 17 | EIVLTQSPGTLSLSPGERATLSCRASQRVSSSYLAWYQHKFGQAP RLLIYGASRRATGIPDRFSGSGSGTDFTLTISRLEPEDFAVYYCQQY GSSPRSFGQGTKLEIK | 18 |
| **D6P7** | EVQLLESGGGLVQPGGSLRLSCAASGFNFRSYGMNWVRQAPGKG LEWVSSLRSSGVTTYYADSVKGRFTISRDNSKNTLYLQMNSLRGE DTAVYYCAKDRDAEWLGSEPFDPWGQGTLVTVSS | 23 | EIVLTQSPGTLSLSPGERATLSCRASQSLSNSYLAWYQHKPGLAPR LLIYDASSRATGIPDRFSGSESGTDFTLTISRLEPEDFAVYYCQQYG SPPRSFGQGTKLEIK | 24 |
| **E4P7** | EVQLLESGGGLVQPGGSLRLSCAASGFSFRSYGMNWVRQAPGKG LEWVSSLRSSGVTTYYADSVKGRFTISRDNSKNTLYLQMNSLRGE DTAVYYCAKDRDAEWLGSEPFDPWGQGTLVTVSS | 26 | EIVLTQSPGTLSLSPGERATLSCRASQSLSNSYLAWYQHKPGQAPR LLIYDASSRATGIPDRFSGSGSGTDFTLTISRLEPEDFAVYYCQQYG SPPRSFGQGTKLEIK | 27 |
| **G5P4** | EVQLLESGGGLVQPGGSLRLSCVASGFSLRGYAMNWVRQAPGRG LEWVSSLTSNGATAYYADSVKGRFTISRDNFKNTLYLQMHSLRGED TAVYYCARDRDAEWLGSEPFDPWGQGTMVTVSS | 32 | EIVLTQSPGTLSLSPGERATLSCRASQSVSSSYLAWYQHKPGQAP RLLIYGASSRAIGIPDRFSGSGSGTDFTLTISRLEPEDFAVYYCQQY GSSPRSFGQGTKLEIK | 33 |
| **B8P4** | EVQLLESGGALVQPGGSLRLSCAASGFTFRSYAMNWVRQAPGKG LEWVSSLSSSGTTTYYADSVKGRFTISRDNSKNTMYLQMDSLRGE DTAVYYCAKDRNAQWLGSEPLDPWGQGTLVTVSS | 38 | EIVLTQSPGTLSLSPGERATLSCRASQRVGSNYLAWYQHKPGQAP RLLIYGASSRATGIPDRFSGSGSGTDFTLTISRLEPEDFAVYYCQQY GSSPRSFGQGTKLEIK | 39 |

### Competing antibodies and epitopes

In a further aspect, the invention provides an antibody, or antigen-binding fragment thereof, that binds and neutralizes a plurality of BK virus subtypes including at least subtype I, II and IV, wherein said antibody (1) competes for binding to a BKV viral protein 1 (VP1), with a reference antibody of the invention as described above, such as antibody E3P7 (VH: SEQ ID NO: 11; VL: SEQ ID NO: 12), D10P4 (VH: SEQ ID NO: 17; VL: SEQ ID NO: 18), D6P7 (VH: SEQ ID NO: 23; VL: SEQ ID NO: 24), E4P7 (VH: SEQ ID NO: 26; VL: SEQ ID NO: 27), G5P4 (VH: SEQ ID NO: 32; VL: SEQ ID NO: 33), and/or B8P4 (VH: SEQ ID NO: 38; VL: SEQ ID NO: 39).

Any BKV viral protein 1 (VP1) as described in above Table 1 may be used herein. A particularly preferred BKV viral protein 1 (VP1) is of SEQ ID NO: 40.

The competition for binding is herein preferably determined in a competition ELISA binding assay, also called cross-competition ELISA. Briefly, for such assay, ELISA plates can be pre-coated with VP1 protein (e.g. SEQ ID NO: 40), which may be in the form of VP1 pentamers and incubated with a labelled reference antibody (at a fixed concentration; the label may be biotin) in (e.g. three-fold dilution; preferably at a starting concentration of 100µg/mL) serially diluted solutions of an unlabeled antibody competitor in a suitable buffer (e.g. PBS). Antibody incubation step can then be performed, preferably for about 1h.

In a preferred embodiment, the antibody (1) inhibits at least 30%, preferably at least 50% or 60%, more preferably at least 70%, 75%, 80%, 85%, 90% or 95% of binding of at least one of the reference antibodies E3P7 (VH: SEQ ID NO: 11; VL: SEQ ID NO: 12), D10P4 (VH: SEQ ID NO: 17; VL: SEQ ID NO: 18), D6P7 (VH: SEQ ID NO: 23; VL: SEQ ID NO: 24), E4P7 (VH: SEQ ID NO: 26; VL: SEQ ID NO: 27), G5P4 (VH: SEQ ID NO: 32; VL: SEQ ID NO: 33), and/or B8P4 (VH: SEQ ID NO: 38; VL: SEQ ID NO: 39) to a BKV viral protein 1 (VP1) (e.g. of SEQ ID NO: 40), as preferably determined in a competition ELISA binding assay as described herein.

In another aspect, the invention provides an antibody, or antigen-binding fragment thereof, that binds and neutralizes a plurality of BK virus subtypes including at least subtype I, II and IV, wherein said antibody (2) binds to an epitope comprising one or more, preferably a plurality of, amino acids selected from P59, D60, E61, L63, F66, L68, K69, N74, F76, D75, F76, S78, E82, H139, F271, T277 and/or S275 in a BKV viral protein 1 (VP1) of SEQ ID NO: 40.

It will be readily understood that any other BKV viral protein 1 (VP1) as described in above Table 1 may be used herein.

The epitope is herein preferably determined by Cryo-Electomicroscopy (cryo-EM), preferably at a contact distance resolution ranging from 0 to 6 Angström. Briefly, for such assay, the VP1 protein, which may be in the form of VP1 pentamers or VLPs, is incubated with the antibody, or antigen-binding fragment thereof, at an optimized ratio (e.g. two-fold molar ratio) to allow the formation of a stable complex. The complex is applied to an EM grid, which is then plunged into a cryogen. Automated image acquisition can be performed on a cryo-EM microscope equipped accordingly, and images processed to reconstruct 3D density map using a suitable algorithm. Atomic models of VP1 and antibody can be fitted into the 3D density map, to analyze the VP1 residues contacted by the antibody or antigen-binding fragment. A local refinement may be performed to improve resolution at the epitope interface. A more detailed protocol is provided in the below Examples, section 1.12.

In a preferred embodiment, the epitope of antibody (2) comprises one or more, preferably a plurality of, amino acids selected from P59, D60, E61, N74, D75, F76, S78, H139 and/or S275 in BKV viral protein 1 (VP1) of SEQ ID NO: 40, as preferably determined by Cryo-Electomicroscopy (cryo-EM) as described herein.

In another preferred embodiment, the epitope of antibody (2) comprises one or more, preferably a plurality of, amino acids selected from P59, D60, E61, L63, F66, L68, K69, F76, E82, F271 and/or T277 in BKV viral protein 1 (VP1) of SEQ ID NO: 40, as preferably determined by Cryo-Electomicroscopy (cryo-EM) as described herein. Preferably the one or more amino acids are selected from D60, E61, K69, F76, E82 and/or T277.

Binding to VP1 can be further analyzed in an ELISA binding assay, in which for example, multiple serial dilutions of those antibodies can be tested in ELISA plates precoated with VP1.

In a further aspect, the invention provides an antibody, or antigen-binding fragment thereof, that binds and neutralizes a plurality of BK virus subtypes including at least subtype I, II and IV, wherein said antibody (3) has reduced binding or loses binding to a mutant BKV viral protein 1 (VP1), said mutant containing one or more, preferably a plurality, of the following amino acid mutations in SEQ ID NO: 40: P59A, D60A, E61A, L63A, F66A, L68A, K69A, N74A, D75A, F76A, S78A, E82A, F271A, T277A, and/or S275A.

It will be readily understood that any other BKV viral protein 1 (VP1) as described in above Table 1 may be used herein.

Binding to VP1 mutants is herein preferably determined in an ELISA binding assay, in which for example, multiple serial dilutions of those antibodies can be tested in ELISA plates precoated with VP1 mutants and the naturally-occurring VP1. Briefly, for such assay, wells of ELISA plates can be pre-coated with either VP1 protein (e.g. SEQ ID NO: 40) or mutants thereof, preferably in the form of VP1 pentamers or VLPs, and incubated with serial dilutions (e.g. threefold dilutions) of antibody (e.g. starting concentration of 50µg/ml) in a suitable buffer (e.g. PBS). Antibody incubation step can then be performed, typically for about 1 h.

In a preferred embodiment, the antibody (3) has a binding to those mutants that is reduced by greater than 30%, preferably greater than 40%, 50%, 60%, more preferably greater than 70%, 75%, 80%, 85%, 90% or 95% relative to the binding between the same antibody and naturally-occurring VP1 (SEQ ID NO: 40).

In a preferred embodiment, the antibody (3) has reduced binding or loses binding to a VP1 mutant containing one or more, preferably a plurality, of the following amino acid mutations in SEQ ID NO: 40: P59A, D60A, , E61A, N74A,D75A, F76A, S78A, H139A and/or S275A, as preferably determined in an ELISA binding assay as described herein.

In another preferred embodiment, the antibody (3) has reduced binding or loses binding to a VP1 mutant containing one or more, preferably a plurality, of the following amino acid mutations in SEQ ID NO: 40: P59A, D60A, E61A, L63A, F66A, L68A, K69A, F76A, E82A, F271A and/or T277A, as preferably determined in an ELISA binding assay as described herein. Preferably the one or more amino acids are selected from D60, E61, K69, F76, E82 and/or T277.

In a preferred embodiment, the antibody (1), (2) or (3) as described above is a human antibody and/or a monoclonal antibody.

### Constant regions

The antibody constant regions, if present, may be selected in line with the intended use of the antibody, notably in line with the effector function(s) required for that use.

In the context of the present invention, constant regions are preferably those of a human antibody, such as a human IgA, IgD, IgE, IgG, or IgM, preferably a human IgG.

When the constant regions are those of a human IgG, the heavy chain (HC) constant region preferably contains a CH1 domain, a CH2 domain and a CH3 domain; and/or the light chain (LC) constant region contains a CL domain, either kappa or lambda.

Human IgG constant regions may be selected from any hIgG isotype, i.e. hIgG1, hIgG2, hIgG3 or hIgG4, depending on the intended use. These isotypes are indeed known to possess unique sequences and properties, with regard to their half-life and effector functions, the latter ranging from complement-dependent cytotoxicity (CDC), antibody-dependent cellular cytotoxicity (ADCC) to antibody-dependent cell phagocytosis (ADCP). These properties are mediated by the Fc region, i.e. by the CH2 and CH3 domains of the antibody's two heavy chains, and ultimately promote the killing of pathogen-infected cells and tumor cells.

For example, the half-life of hIgG3 (e.g. about 5-7.5 days) is typically much shorter than those of hIgG1, hIgG2, and hIgG4 (e.g. about 14-21 days). As another example, hIgG4 has reduced effector functions compared to hIgG1, hIgG2, and hIgG3. hIgG1 and hIgG4 are are also known to be more prone to proteolytic cleavage than hIgG2.

These difference in properties are essentially due to difference in binding affinity of the Fc region to receptors involved in effector functions, namely the Fcγ receptors (FcyRI, FcyRIIA, FcyRIIC and FcyRIIIA), the neonatal Fc receptor (FcRn), and the complement component 1q (C1q). The Fc region of IgG also typically contains a glycosylation site, which is believed to facilitate interaction with those receptors.

Thus, for a therapeutic use, one would favour using constant regions, especially the Fc region, that is best suited based on each isotype specific properties. In the context of the present invention, it shall be understood that selecting hIgG isotypes with strong effector functions is preferred.

These properties can nevertheless be optimized, by modulating for example antibody serum persistence (higher circulating levels, allowing less frequent administration and/or reduced doses), and/or pathogen clearance. Such enhancements may be achieved by introducing mutations and/or post-translational modifications, more particularly in the Fc region.

Thus, in a preferred embodiment, the antibody or antigen-binding fragment thereof according to the invention further comprises, especially in the Fc region, modifications which modulate Fc/FcRn binding, complement binding, complement-dependent cytotoxicity (CDC), antibody-dependent cellular toxicity (ADCC), antibody-dependent cell phagocytosis (ADCP), glycosylation and/or pharmacokinetics such as half-life.

The term "*modulate*" or "*modulation*" generally means inhibit(ion)/diminish or increase/augment(ation). In a preferred embodiment, modulate means increase.

Fc modification strategies and techniques are well-known in the art (Saunders, Front Immunol.. 2019; 10:1296, see e.g. Tables 1-3; Abdeldaim et al., Pharmaceutics 2023; 15(10): 2402; both reviews being incorporated herein by reference). One can, for example, engineer cross-isotype Fc regions (i.e. merge constant domains from different IgG isotypes to engage a wider range of Fc receptors), or introduce specific mutations in Fc regions to modulate effector functions and/or half-life. Sensitivity to proteolytic cleavage may also be abrogated.

Examples of human IgG engineered Fc regions that are suitable in the present invention are illustrated in Table 7 below.

**Table 7. Engineered human IgG Fc regions. Numbering scheme: EU. For correspondence with IMGT, see the IMGT Scientific chart as provided on the IMGT website (e.g. www.imgt.org/IMGTScientificChart/Numbering/Hu_IGHGnber.html#refs).**

| **Engineered Fc** | **Isotype** | **Mutations ["name" of the mutation]** | **FcR/C1q Binding** | **Effector Function** |
|---|---|---|---|---|
| hIeG1b12-Fc | IgG1 | M418L/N434S ["LS"] | Increased binding to FcRn | Increased half-life |
| hIeG1e1-Fc | IgG1 | T250Q/M428L | Increased binding to FcRn | Increased half-life |
| hIuG1e2-Fc | IgG1 | M252Y/S254T/T256E["YTE"] + H433K/N434F | Increased binding to FcRn | Increased half-life |
| hIgG1e4-Fc | IgG1 | E333A | Increased binding to FcγRIIIa | Increased ADCC and CDC |
| hIuG1e5-Fc | IgG1 | S239D/A330L/I332E ["DLE"] | Increased binding to FcγRIIIa | Increased ADCC |
| hIuG1e6-Fc | IgG1 | P257I/Q311 | Increased binding to FcRn | Unchanged half-life |
| hIG1e7-Fc | IgG1 | K326W/E333S | Increased binding to C1q | Increased CDC |
| hIuG1e9-Fc | IgG1 | S239D/I332E/G236A ["DEA"] | Increased FcγRIIa/FcγRIIb ratio | Increased macrophage phagocytosis |
| hIeG4e1-Fc | IgG4 | L234A/L235A ["LALA"] | Increased binding to FcRn | Increased half-life |
| hIeG4e2-Fc | IgG4 | S228P + 252Y/S254T/T256E ["YTE"] | - Increased binding to FcRn | Reduced Fab-arm exchange |
| | | | | Increased half-life |
| hIgG4e3-Fc | IgG4 | | | Abolition of cleavage motif at the Cter of the antibody (hinge) |
| hIgG1e3-Fc | IgG1 | K444A | | |

Post-translational modification of the Fc region by glycoengineering, chemical or enzymatic treatment may also be implemented. For example, galactosylation of IgG1 Fc region can increase C1q binding and CDC activity, as well as thermostability; this improvement is also applicable to IgG3. The single N-linked glycosylation site at position 297 within IgG1 Fc region can also be modified to improve ADCC activity, via e.g. hypofucosylation or afucosylation.

The type and extent of post-translation modification often depends on the host cell used to express the antibody as well as the culture conditions.

Other strategies conventionally used to extend half-life of protein therapeutics may also be applied herein. These include for example conjugation to polymers (e.g. PEGylation, polysialylation), carbohydrates, or serum albumin, to name a few.

### Antibody production

To produce an antibody, or antigen-binding fragment thereof, a nucleic acid or a group of nucleic acids encoding therefore are typically subcloned into one or more suitable vectors. Such vectors can be transfected into host cells, such as mammalian cells, which can then be cultured under appropriate conditions to produce the desired antibody or antigen-binding fragment thereof. Methods for producing antibodies and their antigen-binding fragments are well-known in the art (see e.g. Current protocols in Immunology, pp 10.19.1-10.19.11, Coligan et al. (eds.), Wiley Interscience, 1992; "Antibody engineering: a practical guide" by Borrebaeck Carl, W.H. Freeman and Company, New York, 1992; Immunobiology by Janeway et al., 5th edition, Garland publishing, 2001; Therapeutic Monoclonal Antibodies: From Bench to Clinic by Zhiqiang An, Wiley editions, 2009; Antibodies: A Laboratory Manual, by Harlow et al., Cold Spring Harbor Laboratory, New York, 1988; the relevant excerpts of each the above-mentioned manuals being hereby incorporated by reference in their entirety).

The present invention accordingly extends to a nucleic acid or set of nucleic acids encoding the antibody, or antigen-binding fragment thereof, as described above.

The terms "*nucleic acid*"*,* "*nucleic acid molecule*"*,* "*polynucleotide*" and "*nucleotide sequence*" are herein equivalent and refer to a polymer of nucleotides. These nucleotides may be natural (namely, A, T, G, C and U) or non-natural, preferably natural. These terms encompass a single-stranded or double-stranded DNA, as well as the transcription product of said DNA, such as an RNA. The nucleic acid is herein preferably a double-stranded DNA.

In a preferred embodiment, a nucleic acid may encode the heavy chain variable domain (VH) of the antibody, and another nucleic acid may encode the light chain variable domain (VL) of said antibody, said domains being as described above. More preferably, said nucleic acids are provided herein as a set of nucleic acids.

In a preferred embodiment, when the antibody contains constant regions, a nucleic acid may encode the heavy chain variable domain (VH) of the antibody, another nucleic acid the heavy chain constant domain (CH, which may comprise CH1, CH2 and CH3 domains) of said antibody, another nucleic acid the light chain variable domain (VL) of said antibody, and another nucleic acid the light chain constant domain (CL) of said antibody, said domains being as described above. More preferably, said nucleic acids are provided herein as a set of nucleic acids.

In another preferred embodiment, when the antibody contains constant regions, a nucleic acid may encode the heavy chain variable domain (VH) and the heavy chain constant domain (CH, which may comprise CH1, CH2 and CH3 domains) of the antibody, and another nucleic acid may encode the light chain variable domain (VL) and the light chain constant domain (CL) of said antibody, said domains being as described above. More preferably, said nucleic acids are provided herein as a set of nucleic acids.

The nucleic acid or set of nucleic acids according to the invention can be prepared by methods well-known in the art, including, but not limited to, any synthetic and/or recombinant method such as *de novo* solid phase DNA synthesis or by PCR mutagenesis of an existing anti-BKV antibody. It is within the skill of the person in the art to design the nucleotide sequence of a nucleic acid so as to allow an efficient production of the antibody, such as by codon-optimization based on the desired expression system (e.g. host cell).

The nucleic acid or set of nucleic acids according to the invention can be comprised in a vector capable of expressing the antibody, or antigen-binding fragment, in a suitable host cell. Said nucleic acids can thus be cloned either in separate vectors, or, in a single vector.

The invention therefore further relates to a vector, preferably an expression vector, which comprises the nucleic acid or set of nucleic acids as disclosed herein.

The term "*vector*" refers herein to a molecule, typically a nucleic acid molecule, used as a vehicle to transfer genetic material, such as a nucleic acid of interest, typically into a cell. Such vectors are very well known in the art, and include vectors capable of replication in order to amplify a nucleic acid of interest (i.e. a cloning vector), and/or to express the polypeptide encoded by said nucleic acid in a host cell (i.e. an expression vector). These types of vectors are publicly available and encompass for example plasmids, cosmids, artificial chromosomes (e.g. YACS, BACS), viral vectors (adenovirus, AAV, retrovirus such as lentivirus, EBV episome, etc.), and phage vectors. A vector is said to be recombinant when it is not found in nature, such as when it includes an heterologous nucleic acid of interest (i.e. it is not naturally-occurring). In general, vectors comprise at least an origin of replication, a multicloning site for incorporating a nucleic acid of interest, and one or more selection markers (e.g. antibiotic resistance), along with a larger sequence that serves as a backbone. Additional features of vectors typically include, without limitation, enhancers, promoters, protein purification tags, and/or a secretory signal peptide sequence (for the polypeptide of interest to be secreted into the culture medium). Should the nucleic acids coding for the heavy and light chains be included into a single vector, their sequences may be separated by a polyadenylation signal, and their transcription be driven by the same or different promoters, preferably by different promoters. The choice of the vector, and its features, will depend on the intended host cells in which it will be introduced.

The invention further pertains to a host cell expressing the antibody, or antigen binding fragment thereof, or comprising the nucleic acid, set of nucleic acids, or vector (or set of vectors) as disclosed herein.

The terms "*host cell*", "*cell*" and "*cell line*" can be used interchangeably to refer to a prokaryotic or eukaryotic cell in which a nucleic acid or vector can be introduced, such as for amplification of the nucleic acid of interest, and/or to express the polypeptide encoded by said nucleic acid. To this end, a host cell may be "*transfected*" or "*transformed*" by a process known in the art by which said nucleic acid or vector is transferred or introduced into the host cell. Examples of such methods include, without limitation, electroporation, lipofection, calcium phosphate transfection, transfection using DEAE dextran, microinjection, and the like. It should be understood that the term "*host cell*" also encompasses any progeny thereof which retains all the essential features of the parent host cell.

Techniques for inserting a nucleic acid into a vector and/or transferring a nucleic acid or vector into a host cell, are detailed in Molecular Cloning: A Laboratory Manual (4th edition) by Sambrook et al., Cold Spring Harbor Laboratory Press, 2012.

The skilled person will be able to choose the appropriate host cell among the many cell lines that are publicly available, notably among those suited for producing antibodies (see e.g. Yazaki et al. pages 255-268 in Antibody Engineering: Methods and Protocols by Benny K. C. Lo, pages 255-268, Humana Press, Totowa, NJ 2004). Eukaryotic cells are herein particularly preferred, and include mammalian cells, plant cells, insect cells or yeast cells. Mammalian cells are herein especially favored, for their capacity to provide suitable post-translational modifications such as glycosylation.

Non-limiting examples of mammalian host cells that can be used according to the invention include, without limitation, Chinese hamster ovary cells (CHO) and derivatives thereof (such as DHFR⁻ CHO cells), baby hamster kidney cells (BHK), human embryonic kidney cells (HEK 293), human embryonic retinal cells (PERC.6), human cervical carcinoma cells from Henrietta Lacks (HeLa), human lung cells (W138), human fetal lung fibroblast cells (MRC-5 cells), human liver cells (Hep G2), human foreskin cells (FSA), human myeloma cells (Y0, also referred as YB2/3.0Ag30), African green monkey kidney cells (Vero, such as VERO-76), monkey kidney cells (CV1) or derivatives thereof (COS cells, COS-7 cells), mouse Sertoli cells (TM4), mouse mammary tumor cells (MMT 060562), murine myeloma cells (NS0, Sp2/0), buffalo rat liver cells (BRL 3A), and canine kidney cells (MDCK).

Particularly preferred mammalian host cells according to the invention are CHO cells, HEK 293 cells, COS cells, or NS0 cells, more preferably CHO cells or HEK 293 cells, even more preferably CHO cells.

The present invention also provides a method for producing the antibody, or antigen-binding fragment thereof, as described above, said method comprising a) culturing the host cell under conditions allowing the expression of said antibody, or antigen-binding fragment thereof, and b) recovering said antibody, or antigen-binding fragment thereof.

In step a), the host cell may be cultured in any appropriate culture medium, which enables the expression of said antibody, or antigen-binding fragment thereof. Such culture media are well-known in the art, and need not be detailed herein.

In step b), the antibody, or antigen-binding fragment thereof, may be recovered from the host cell if said antibody, or antigen-binding fragment thereof, is expressed intracellularly.

Alternatively, in step b), the antibody, or antigen-binding fragment thereof, may be recovered from the culture medium in which the host cell is cultured if said antibody, or antigen-binding fragment thereof, is expressed extracellularly.

The antibody, or antigen-binding fragment thereof, recovered in step b) can advantageously be purified, in a further step of said method, defined as step c). Preferably, said purification step allows the obtention of a 100%-purified or almost 100%-purified antibody, or antigen-binding fragment thereof

The skilled person in the art may use any conventional method known in the art allowing the purification of a polypeptide, such as by chromatography.

### Composition, combination, kit and uses thereof

The invention also relates to a pharmaceutical or diagnostic composition comprising (1) at least one of the antibody, or antigen-binding fragment thereof, as described above, or any combination thereof; and (2) optionally at least one pharmaceutically or diagnostically acceptable excipient.

As used herein, the term a "*acceptable excipient*" means an inactive or inert, and therefore nontoxic compound. A "*pharmaceutically acceptable excipient*" is typically an acceptable excipient of pharmaceutical grade that is devoid of pharmacological action itself, and is accordingly physiologically compatible. A "*diagnostically acceptable excipient*" is typically an acceptable excipient that does not interfere in the signal to be detected in a diagnostic assay. Pharmaceutically or diagnostically acceptable excipients can be used to improve properties of a composition, such as shelf-life, stability, or retention time or consumer acceptance if administered to a subject, etc. Examples of pharmaceutically or diagnostically acceptable excipient include, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like.

It will be understood that the pharmaceutically or diagnostically acceptable excipient is preferably present in the composition in an amount effective to preserve the binding and neutralizing activities of the antibody or antigen-binding fragment thereof according to the invention.

The composition according to the invention may be in a solid form (such as in the form if a powder or tablet), or in a liquid form (such as in the form of a solution or aerosol).

The composition according the invention can be used in several industrial applications, for *in vivo* or *in vitro* use, more particularly in medical or diagnostic applications.

The pharmaceutical composition according the invention can notably be used to treat a BKV and/or a JCV infection, or any related affection thereof.

The invention thus provides a pharmaceutical composition according to the invention for use as a medicament, preferably for the treatment of a BK virus (BKV) and/or a JC virus (JCV) infection or any associated disorder thereof in a subject in need thereof, such as an immunocompromised subject, especially a graft-recipient such as a kidney-graft or bone marrow-graft recipient.

The invention is also directed to the use of the pharmaceutical composition according to the invention, for the preparation of a medicament, preferably for the treatment of a BK virus (BKV) and/or a JC virus (JCV) infection or any associated disorder thereof in a subject in need thereof, such as an immunocompromised subject, especially a graft-recipient such as a kidney-graft or bone marrow-graft recipient.

The invention also provides a method for treating a BK virus (BKV) and/or a JC virus (JCV) infection or any associated disorder thereof, in a subject in need thereof, such as an immunocompromised subject especially a graft-recipient such as a kidney-graft or bone-marrow-graft recipient, said method comprising the administration to the subject of a therapeutically effective amount of the pharmaceutical composition according to the invention.

Typical examples of BKV- of JCV-associated disorders are as described above. A particularly preferred BKV-associated disorder is a BKV-related leukodystrophy.

In the context of the present invention, it will be understood that the subject to be treated, such as an immunocompromised subject, especially a graft-recipient such as a kidney-graft or bone marrow-graft recipient, is preferably a human.

The invention further relates to a pharmaceutical composition as described herein, and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus (JCV).

It shall be understood that when a plurality of antibodies or antigen binding fragments thereof are to be administered to the subject, those may not necessarily be provided in a single pharmaceutical composition.

Accordingly, the invention further pertains to a combination of two or more antibodies or antigen binding fragments thereof, as described herein.

The invention is further directed to two or more antibodies or antigen-binding fragments thereof, as a combined preparation for simultaneous, separate or sequential use as a medicament, preferably in the treatment of a BK virus (BKV) infection or associated disorder thereof, in a subject in need thereof, such as an immunocompromised subject, especially a graft-recipient such as a kidney-graft or bone marrow-graft recipient.

The pharmaceutical composition or antibodies according to the invention can be administered to the subject in need using any suitable scheme of administration. For instance, the administration may be oral, intravenous, subcutaneous, dermal, intradermal, intramuscular, mucosal, parenteral, intraorgan, intralesional, intranasal, inhalation, intraocular, intramuscular, intravascular, intrathecal, intranodal, rectal, intraperitoneal, or any one or combination of a variety of well-known routes of administration, depending on the infection or associated disorder affecting the subject. The route of administration may also depend on whether local or systemic treatment is desired. The dose and/or scheme of administration can be easily determined and adapted by the skilled practitioner, in accordance with the age, weight and/or severity of the infection or associated disorder from which the subject suffers.

In a preferred embodiment, the pharmaceutical composition or antibodies are in a form suitable for a sustainable release, and/or for intravenous, intramuscular, subcutaneous, intranasal, or intrathecal administration, or for administration by infusion or by aerosol.

The diagnostic composition of the invention may be used to detect *in vitro* BKV and/or JCV, which may be particularly useful when performing a graft on a patient.

The invention accordingly provides an *in vitro* use of a diagnostic composition according to the invention, for detecting the presence of BKV and/or JCV in a biological sample.

A "*biological sample*" refers herein to a tissue or cell sample isolated from a subject to be tested, that is likely or suspected to be infected with BKV and/or JCV. In the context of a graft, the subject to be tested may be the graft-recipient and/or the graft-donor. Biological samples susceptible to contain BKV and/or JCV are typically renal samples (e.g. renal biopsies), urine samples, blood samples, or bone marrow samples.

The invention is also directed to a method for detecting the presence of BKV and/or JCV in a biological sample, said method comprising contacting *in vitro* the biological sample with the diagnostic composition according to the invention.

More precisely, the invention is directed to an *in vitro* method for detecting the presence of BKV and/or JCV in a biological sample, said method comprising: contacting said sample with the diagnostic composition, and detecting the binding of the antibody or antigen-binding fragment to a BK virus, and/or detecting neutralization of a BK virus by the antibody or antigen-binding fragment.

The invention is also directed to a method for diagnosing a BKV and/or JCV infection or associated disorder thereof in a subject, said method comprising contacting *in vitro* a biological sample of the subject with the diagnostic composition according to the invention.

More precisely, the invention is directed to an *in vitro* method for diagnosing a BKV and/or JCV infection or associated disorder thereof in a subject, said method comprising: contacting said sample with the diagnostic composition, and detecting the binding of the antibody or antigen-binding fragment to a BK virus, and/or detecting neutralization of a BK virus by the antibody or antigen-binding fragment.

The contacting step is preferably performed under conditions allowing the binding between VP1 and the antibody, or antigen-binding fragment thereof, that is contained in the composition. The detection of the binding is accordingly indicative of the presence of BKV and/or JCV in the biological sample, and/or that the subject is suffering from a BKV and/or JCV infection or associated disorder thereof. Alternatively and/or additionally, the detection of neutralization is indicative of the presence of BKV and/or JCV in the biological sample and/or that the subject is suffering from a BKV and/or JCV infection or associated disorder thereof.

The binding can be detected by any methods well-known in the art, such as by Western-Blot, RIA, or ELISA, to name a few. To facilitate the detection of binding, the antibody, or antigen-binding fragment thereof, that is contained in the diagnostic composition may be labeled, notably with a detectable molecule, such as with a radiolabel, an enzyme label, a fluorescent label, a biotin-avidin label, a chemiluminescent label, and the like.

The neutralization can be detected by any methods well-known in the art, such as pseudotype virus assay, microneutralization assay, pseudotype virus assay, plaque reduction assay, rapid lateral flow, and the like.

The present invention also pertains to a kit for implementing any method as described herein, said kit comprising: (1) an antibody, or antigen-binding fragment thereof, or a pharmaceutical or diagnostic composition, or a nucleic acid, or a set of nucleic acids, or a vector (or set of vectors), or a host cell, as described above, or any combination thereof; and (2) instructions for implementing the method.

The term "*instructions*" refers to written, printed, or graphic displays generally provided on or alongside a container of a kit, for example written material presented on a bottle containing an active agent (herein (1)), such as details of its content and use thereof.

The present invention finally pertains to an *in vitro* use of anti-idiotypic antibody that binds to the antibody or antigen-binding fragment thereof as described herein, for measuring the concentration of said antibody or fragment in a sample, preferably in a biological sample..

The present invention will be better understood in the light of the following detailed experiments. Nevertheless, the skilled artisan will appreciate that the present examples are not limitative and that various modifications, substitutions, omissions, and changes may be made without departing from the scope of the invention.

### EXAMPLES

In the present study, specific memory B cell sorting followed by single cell PCR were used to isolate anti-BKV human monoclonal antibodies from donors' blood. By using labelled antigens, specific single memory B-cells were isolated by flow-cytometry. Cloning and expression of human monoclonal antibodies were achieved through the recovery of immunoglobulin gene sequences from single B cells and by transfection of producer cells with the VH and VL chain genes. Recombinant BKV capsid protein VP1 of the three different BKV genotypes I, II and IV and of viral variants thereof, were used as antigen. Lead antibody candidates were selected for their binding and broad neutralization potency, and their respective epitopes characterized.

### 1. MATERIALS AND METHODS

### 1.1. Donor material

Human peripheral blood (PBMCs) were obtained and collected from 18 donors after receiving their written informed consent and approval by the Institutional Review Boards of the Strasbourg University Hospitals. PBMCs were isolated and stored in liquid nitrogen. Elite BKV neutralizers were identified as those having high serum titers (e.g. > 1/10⁵ IC₅₀) of anti-BKV Abs against all tested BKV genotypes.

### 1.2. Cell Culture

BL21 Star strain of *Escherichia coli* cells (Invitrogen, #C6010) used for VP1 pentamers production, were cultured at 37°C and 26°C in LB medium supplemented with 50 µg/mL of ampicillin. Human monoclonal antibodies and Fabs were expressed in HEK293F cells cultured in FreeStyle 293 expression medium (Thermo-Fisher, #12338018) at 37°C under 5% CO2. 293TT cells (National Cancer Institute Tumor repository, Frederick, Maryland) were cultured as previously described by Solis et al. (J Am Soc Nephrol., 2018; 29(1):326-334).

### 1.3. VP1 pentamers

### Plasmids construction

A truncated form of VP1 from BKV genotype I was cloned into a pET-15b expression plasmid. This truncated form contains the amino acid residues 31 to 301, and is referred as the Dunlop sequence except for the cysteine residue at position 104 that is replaced by a serine (Neu et al. PLoS Pathog, 2013; 9: e1003688). VP1 of genotype II and IV were obtained by gene synthesis (Eurofins) and sub-cloned into a pESPRIT002 vector which contains a His-tag in N-Ter and a biotin acceptor peptide (*BAP*) in C-Ter. VP1 of genotype I was also subcloned into a pESPRIT002 plasmid.

### Production and purification of VP1 pentamers

VP1 pentamers were produced *in E.coli* and purified through a His tag on a nickel column. To do so, BL21 Star *E.coli* were firstly transformed with the pET15-b plasmid expressing the truncated VP1, and cultured until reaching an OD of ~0.6. After IPTG induction, cells were grown at 26 °C for 18 h. After centrifugation, the cell pellet was resuspended in buffer A (50 mM Tris-HCl, pH 7.5, 250 mM NaCl, 30 mM imidazole, 5% glycerol, complete EDTA - free protease inhibitor cocktail and sonicated. The bacterial lysate was pelleted by high speed centrifugation while the supernatant was filtered and applied onto a Ni-NTA resin (Ni-NTA superflow Qiagen, ref 30410), equilibrated with buffer A. Resin was washed with buffer A and VP1 pentamers were eluted in different fractions from the column with buffer B (50 mM Tris-HCl, pH 7.5, 250 mM NaCl, 500 mM imidazole, 5% glycerol). Fractions corresponding to pentamers, identified on Coomassie-stained SDS-PAGE, were pooled together and dialyzed. The sample was then concentrated using a 50,000 molecular weight cut-off (MWCO) Amicon concentrator and VP1 pentamers were purified using a Superdex increase S200 column (GE Healthcare) in 20 mM Hepes pH 7.5, 150 mM NaCl buffer, using an AKTA Purifier (GE Healthcare). Peak fractions were pooled and then concentrated with Amicon concentrator. Quantification of purified pentamers was determined by spectrophotometric absorbance at 280 nm. Pentamers were aliquoted and stored at -80 °C until use.

### Biotinylation of VP1 pentamers

VP1 pentamers were biotinylated using the Biotin-Protein Ligase-BirA-RT kit (Avidity, LLC), according to the manufacturer's instructions. Briefly, 100 µM of VP1, containing a Biotin Acceptor Peptide sequence, was incubated with the BirA enzyme in supermix buffer for 1h at room temperature. The biotinylated protein was then purified by size exclusion chromatography using a Superdex increase S200 column (GE Healthcare) in 20 mM Hepes pH 7.5, 150 mM NaCl buffer, using an AKTA Purifier (GE Healthcare).

### 1.4. Pseudovirions

### Reporter plasmids construction

Plasmids encoding synthetic codon-modified VP1, VP2 and VP3 capsid proteins of BK virus (BKV) were provided by Christopher B. Buck (National Cancer Institute, National Institute of Health, Bethesda, Maryland). The *Gaussia* luciferase (GLuc) reporter plasmid phGluc was constructed by transferring a fragment of the pCMV-Gluc 2 vector (New England BioLabs) into ph2m. Similar plasmids were constructed for production of JCV pseudovirions.

### Pseudovirion production

BKV pseudovirions were produced as previously described by Pastrana et al. (PLoS Pathog. 2012; 8(4): e1002650), for the three BKV genotypes Ia (BK-D; JF894228), BKV II (GBR-12; AB263920) and BKV IVc2 (A-66H; AB369093) as well as for single-or double-mutants thereof (BKV Ia: D60N, L68Q, A72VE73Q, E73Q, D75N, E82D; BKV IVc2: D77Q). Briefly, expression plasmids encoding the VP1, VP2 and VP3 capsid proteins, along with the reporter plasmid phGluc, were co-transfected into 293TT cells. Two days after transfection, cells were lysed and pseudovirions maturated overnight using Ambion^{®} RNase Cocktail^{™} (ThermoFischer Scientific). Pseudovirions were harvested and purified by ultracentrifugation through a iodixanol step gradient (Optiprep, Sigma-Aldrich). To minimize any variation, a single pseudovirion stock was produced for each genotype, aliquoted and used for all experiments.

JCV pseudovirions were produced as previously described by Ray et al. (Sci. Transl. Med 2015; 7(306):306ra151). Briefly, *Gaussia* luciferase reporter genes were packaged into pseudovirions in 293TT cells transiently transfected with JCV VP1/2/3 expression plasmids. The resulting pseudovirions were purified over Optiprep gradients.

### 1.5. Entire replicative virions

Entire replicative virions of BK virus genotype Ia (Dunlop strain ; ATCC VR-837TM), and BKV genotype II and IV (patient's isolates) were produced.

### 1.6. Serum adsorptions for selection of elite BKV neutralizers

Serum adsorptions with VP1-coupled beads were performed using DynaBeads MyOne Tosylactivated kit (Invitrogen, # 65501) according to the manufacturer's instructions. Beads coupling was performed at a ratio of 0.5 mg of VP1 genotype I pentamer per 12.5 mg of beads. Briefly, VP1 pentamers were incubated with beads for 24h at 37 °C. Protein-coated beads were then collected by magnetic force and resuspended in a PBS blocking buffer for another 24h at 37°C. The magnetic beads were then washed 3 times in a PBS washing buffer suspended in DMEM 10% FBS PSG NaPy and incubated 30 min at room temperature. Patients sera or IgG (40 µl) were then added and incubated 30 min at room temperature. After 3 washes, the antibodies bound to beads were eluted by 3 cycles of glycine pH 2.7 followed by 3 cycles of glycine pH 2.2. Each eluted fraction was neutralized by Tris pH 9. Beads were then regenerated and three rounds of adsorption were performed to ensure complete removal of antigen-specific antibodies. The percentage of IgG titer in depleted fraction was calculated by the formula: (IgG titer-depleted/IgG titer-before) × 100%, where "IgG titer-depleted" represents the VP1-specific IgG titer of the patient's serum after the specific antibody depletion, and "IgG titer-before" represents the VP1-specific serum IgG titer before depletion. The percentage of depletion corresponds to : 100% - the percentage of IgG titer in depleted fraction.

### 1.7. Memory B cell sorting with VP1 pentamers

FACS sorting of cryopreserved PBMCs from donor 16 was performed on a BD FACSAria^{™} Fusion cytometer. PBMCs were stained with live/dead fixable Aqua Dead Cell Stain Kit (Thermofisher, #L34957). The following cocktail of antibodies from Miltenyi Biotec were also used to stain the cells prior to sorting by flow cytometry: anti-CD3-BV421, anti-CD19 (#130113649), anti-CD20-PECy7 (#130111345), anti-Human IgA-PE (#130113476), anti-IgM-PE (#130093075), anti-IgD-PE (#130110643). VP1 pentamers sorting baits were pre-complexed with a streptavidin fluorophore prior to addition to cells. Five fluorophore were used: Strep-APC (Miltenyi Biotec, #130-106-792) for genotype I, Strep-BUV 496 (BD Biosciences, #612961) for genotype II, =Strep-Vio 515 (Miltenyi Biotec, #130-107-459) for genotype IV, Strep-BUV 395 (BD Biosciences, #564176) for the S275A VP1 mutant, and Strep-BUV 737 (BD Biosciences, #612775) for the Y169A-R170A VP1 mutant. Live CD3/CD8- CD14- CD19+ IgG+VP1+ cells were sorted into individual wells of 96-well PCR plates (free of DNase and RNase, Bio-Rad) containing a lysis buffer. The plates were then stored at -80 °C for further processing.

### 1.8. Neutralization assays using pseudovirions or entire replicative virions

BKV or JCV pseudovirion neutralization was determined by analysis of luciferase reporter gene expression as previously described by Pastrana et al. (PLoS Pathog. 2012; 8(4): e1002650). Briefly, pseudovirions mixed with serially diluted sera or antibodies were added to 293TT cells for 72h at 37°C. Neutralization titers were determined by analysis of luciferase activity using the Gaussia Luciferase Glow Assay Kit (Thermo Fisher Scientific) and a luminescence reader (Victor Novo, Perkin Elmer).

For entire virions neutralization assay, BKV replicative virus was mixed with serially diluted antibodies and added to MRC5 cells for 72h at 37°C. Neutralization titers were determined by VP1 immunostaining using the M02 BKVP1 monoclonal antibody (clone 3B2; Abnova, # MAB3204-M02) and a peroxydase labeled anti-mouse IgG antibody (pi2000 Vector Laboratories) or by PCR quantification of BKV DNA (BKV R-gene^{™} kit, Biomérieux).

The neutralization titer was defined as the sample dilution that yielded 50% inhibition of pseudovirion or entire virion infectivity (IC₅₀) and was expressed as the log10 of the IC₅₀. IC₅₀ values were determined by Graphpad Prism using non-linear regression (curve fit) (GraphPad Software 10.0.2).

### 1.9. Antibodies

### Cloning of antibody variable regions

The RNA extracted from the sorted single B cells was converted into cDNA by RT-PCR using the SuperScript^{™} IV enzyme (Fischer Scientific, # 15317696) and random hexamers (Fischer Scientific, # 10580091). To efficiently and specifically amplify antibody genes from single B cells, two successive PCR reactions were run with different sets of primers. The first set of primers was designed to anneal upstream of the second set. After sequencing and lineage attribution by IMGT, the cDNAs of the first-round nested PCR were used to amplify the human antibody variable regions of heavy and kappa/lambda chains for subsequent cloning using appropriate primers with overlapping sequences. PCR products were purified and cloned by the overlapping sequences into human-IgG (Heavy, Kappa or Lambda) expression plasmids using the In-Fusion HD enzyme, according to the manufacturer's instructions (In-Fusion HD Cloning Plus kit, Takara Bio USA, # 638910).

### Monoclonal antibody production and purification

For 100 ml-scale monoclonal antibody production, heavy and light chain-encoded plasmids were re-amplified, and re-sequenced over the entire immunoglobulin Open Reading Frame. A total of 60 µg DNA (light and heavy chain plasmids) was transfected into HEK293F mammalian cells. Cells were cultured in suspension for several days at 37°C before supernatant harvesting and sterile filtration. Antibodies secreted in supernatant were purified via affinity chromatography onto protein A according to the manufacturer's instructions (rProtein A Sepharose^{™} Fast Flow Affinity Media, Cytiva, # 17127903).

### Fabs production and purification

The Fab part of the heavy chain of monoclonal antibodies was obtained after mutagenesis using the "In Fusion HD Cloning Plus" kit (In-Fusion HD Cloning Plus kit, Takara Bio USA, # 638910), according to manufacturer's instructions. The primers designed for the mutagenesis overlapped with the constant part of the heavy chain in order to create a stop codon at amino acid residue K222 corresponding to the Fab part of an antibody. Fabs were produced according to the same procedure as the monoclonal antibodies, , albeit with a prior optimization of the HC/LC chains ratio for transfection. After several days of culture, the Fabs were purified on a Kappa or Lambda Select column (Cytiva, #17545811, # 17548211).

### Mutated antibodies

The LS mutation was introduced into monoclonal antibodies by site-directed mutagenesis.

### Prior art antibodies

Anti-BKV human monoclonal antibodies were used for comparison purpose, namely MTX005 from Memo Therapeutics AG (referred as 319C07 in WO2021/250097), a MTX005 variant (referred as 319C7-var1 in WO2021/250097), and P8D11 from Novartis AG (WO2017046676).

### 1.10. Antibody affinity

### Enzyme-linked immunosorbent assay (ELISA)

Purified VP1 pentamers of genotypes I, II or IV (100 ng/well) were coated on Nunc MaxiSorp 96-well plates (Thermo Scientific # 442404) at 4°C overnight in 150 mM NaCl, 20 mM Hepes pH 7.5 buffer. The following day, plates were washed 5 times with PBST (PBS + 0.1 % Tween 20) and incubated for 1h30 with blocking buffer (10 mM Tris pH7.4,150 mM NaCl,1 mM CaCl2, 4% BSA, 0.1% Tween 20) at 37°C. After removal of the blocking buffer, monoclonal antibodies were added at threefold dilutions (starting at 50 µg/mL) and incubated for 1h30 at 37°C. After washing, an Alkaline Phosphatase-AffiniPure Goat Anti-Human IgG, F(ab')2 Fragment Specific (Interchim, #109-055-097) was added at 1:10,000 dilution for an additional 1h at room temperature. After incubation and 5 wash cycles, the staining substrate (alkaline phosphatase substrate p-nitrophenyl phosphate, (Biorad, #BUF044B) was added for color development. Absorbance at 405 nm was measured after 1h using the TECAN microplate reader (SPARK 10M TECAN # 30086375). Results were analyzed with program GraphPad Prism v9.0 (GraphPad Software) to determine the EC₅₀.

### Biolayer interferometry (BLI)

The kinetics of monoclonal antibodies binding to VP1 pentamers were analyzed using biolayer interferometry (BLI) via a PALL/ForteBio OctetRED96e instrument . Briefly, biotinylated VP1 pentamers (0.5 µg/mL) were captured on streptavidin Capture Biosensors (Sartorius, #18-5020) in loading buffer (150 mM NaCl, 20 mM Hepes pH 7.5, 0,02% Tween 20) until reaching a signal of 1 nm. After capture, the sensors were washed with PBS-T buffer (phosphate buffer saline; 0,0 2% Tween 20) and then submitted to 3 cycles of regeneration (glycine pH 3/PBS-T) for 30s to homogenize the VP1 sample and put in PBS-T buffer during 2 minutes to establish a baseline signal. Then, the VP1 pentamers were placed in wells containing monoclonal antibody at serial concentrations ranging from 0 to 40 nM in loading buffer to determine the association rate. Finally, the sensors were placed in loading buffer wells to test the (off-rate) dissociation rate. Equilibrium dissociation constant (K_{D}) values were generated from the ratio of off-rate values to on-rate values. Data were analyzed through the ForteBio Data Analysis software 7.0 (Molecular Devices LLC).

### 1.11. Antibody auto and polyreactivity

Antibody autoreactivity was tested using the NOVA Lite DAPI ANA kit (Inova Diagnostics, # 704320), according to the manufacturer's instructions. Briefly, monoclonal antibodies were diluted to 100 µg/mL in PBS and incubated on HEp-2 slides. After 30 minutes incubation, slides were washed in PBS, stained with anti-human IgG coupled to FITC and DAPI, and further washed before mounting the coverslips. Pictures were automatically taken using the NOVA View software, but also analysed in parallel by a trained technician using an Olympus BX41 microscope, x40 magnification. Antibody polyreactivity was assessed by ELISA on a panel of antigens including flagellin, insulin, hemo, LPS, lysozyme, thyroglobulin, peptidoglycan, dsDNaA MAPK14 and GP120 (JRFL).

### 1.12. Electron microscopy

### Data collection and processing

To form antigen-antibody complexes, VP1 pentamers or VLPs (the latter being obtained from D.McIlroy, Nantes) were incubated with Fab at a two-fold molar ratio for 1h at room temperature. To eliminate free Fab, the complex was submitted to gel filtration onto S200 increase column. The peak fractions corresponding to the eluted complex were pooled, concentrated (1mg/mL) and applied onto a grid.

### Negative staining

4 µL of sample, diluted to approximately 0.05-0.1 mg/mL, were adsorbed onto the clean side of a carbon film that had been previously evaporated on mica. The sample was then stained with 2% w/v Sodium Silico Tungstate for 30 seconds. The sample and carbon film ensemble was transferred to an EM grid and air-dried. Images were acquired using a Tecnai 12 FEI electron microscope operated at 120 kV, with a Gatan ORIUS SC1000 camera (Gatan, Inc., Pleasanton, CA) at 30,000x nominal magnification.

### Cryo-EM

A 3.5 µL aliquot of the concentrated sample was applied to negatively glow-discharged R1.2/1.3 Quantifoil copper grids (Quantifoil Micro Tools) using a discharge of 25 mA for 40 seconds. Excess solution was blotted away with a Vitrobot Mark IV (FEI) at 20 °C and 100% humidity, with a blot time of 5.5 seconds and blot force set to 0, followed by rapid freezing in liquid ethane. Automated data collection was performed on a 200 kV Glacios cryo-TEM microscope (Thermo Fisher Scientific) equipped with a K2 direct electron detector (Gatan) using SerialEM software. Coma and astigmatism corrections were also performed via SerialEM. Movies consisting of 40 frames were collected in counting mode at a magnification of 36,000×, resulting in a pixel size of 1.145 Å. The defocus range was set between -1.0 and -2.5 µm using a multi-shot scheme (3 × 3 grid of holes without stage movement). The total exposure dose per movie was 40 e-/Å², and approximately 2500 images were obtained per sample.

### Image processing and 3D reconstruction

Movie drift correction and CTF determination were performed using Relion. Particle selection and analysis, including class averaging, initial 3D model generation, and 3D refinement with either I4 or C5 symmetry, were performed with Relion. The final reconstruction resolution was validated by Fourier Shell Correlation using the 0.143 threshold criterion.

### 2. RESULTS

### 2.1. Selection of elite BKV neutralizers

In order to isolate BKV broadly neutralizing monoclonal antibodies, a cohort of kidney transplant patients with strong serum neutralizing activity against the three most prevalent BKV genotypes (I, II and IV) as described by Solis et al. (J Am Soc Nephrol., 2018; 29(1): 326-334) was investigated to select candidates for BKV neutralizing antibodies isolation. The target for BKV neutralizing antibodies is the VP1 capsid protein which forms the outer layer of the virus and whose external surface is accessible to antibodies. The VP1 protein assembles into a pentamer and 72 pentamers form a viral capsid. Recombinant soluble pentamers were used as baits to select specific B cells, in order to isolate neutralizing antibodies. Because such pentamers would not be effective for isolating VLP-specific potential quaternary neutralizing antibodies, serum antibodies were firstly absorbed onto VP1 pentamers from genotype I so as to verify that most neutralizing antibodies could be captured. This approach was used to determine whether VLP-specific quaternary antibodies constitute a significant proportion of the neutralizing activity in the donors, and whether donors with broad serum activity possessed broadly neutralizing antibodies rather than a combination of genotype-restricted antibodies. A loss of neutralization against all three genotypes after adsorption on VP1 from genotype 1 is indeed indicative of the presence of cross-reactive neutralizing antibodies.

In all donors tested (18/18), more than 98% of neutralizing activity against genotype 1 BKV was lost upon sera adsorption, showing that VLP-specific quaternary antibodies do not constitute a significant fraction of the serum neutralizing activity (data not shown). Adsorbed sera were further tested in neutralization against BKV from genotype II and IV, showing a similar loss of neutralizing activity (greater than 94%) in all donors except one (donor 2) (data not shown). Thus, while most donors possessed broadly neutralizing antibodies, the serum activity of some donors was actually due to antibodies that were genotype-specific. Donor 2 was therefore excluded for the isolation of BKV broadly neutralizing antibodies, and donor 16, who exhibited very strong neutralizing titers against the 3 genotypes, was selected for this purpose.

### 2.2. Single memory B cell sorting and production of VP1-specific monoclonal antibodies

Single live CD19+ memory B lymphocytes that bound labeled VP1 pentamers were sorted by FACS from the PBMCs of donor 16 as described above. A total of 600 of these B cells were obtained and seeded at one cell per well in 96-well plates. After cell lysis, RT-PCR, PCR and nested PCR, a total of 349 heavy chain immunoglobulin genes, 228 light kappa genes and 140 light lambda chain genes were recovered. Paired heavy and light chains originating from a same sorted cell corresponded to 239 potential monoclonal antibodies. After sequencing of PCR products, antibody gene usage was assigned using IMGT/V-QUEST (Brochet et al., Nucleic acids research, 2008; 36: W503-508).

The heavy and light chain genes of selected B cells were then further amplified using primers corresponding to the determined gene segment family usage and cloned into the appropriate expression vector (heavy, kappa, lambda). The plasmid DNA of each chain was recovered after transformation in *E. Coli* followed by DNA extraction and transfected in 293F cells. 53 unique monoclonal antibodies were produced, among which 33 had a kappa light chain and 20 had a lambda light chain.

### 2.3. Antibody affinity and germline lineage characterization

The 53 monoclonal antibodies that were produced and purified were subjected to an initial screening to evaluate their binding to BKV VP1. The apparent affinity of each monoclonal antibody for VP1 pentamers of each genotype I, II and IV was assessed by ELISA, as described above. Among them, 6 monoclonal antibodies belonging to the same germline lineage demonstrated a high apparent affinity for VP1 of the 3 genotypes **(Table 8),** and were therefore selected for further studies. Their lineage employed the VH3-23 and JH5 gene segments for the heavy chain, and the VK3-20 and JK2 gene segments for the light chain **(Table 9).** No remarkable feature was noted, with a 16 amino acid HCDR3 and a relatively modest level of somatic hypermutation.

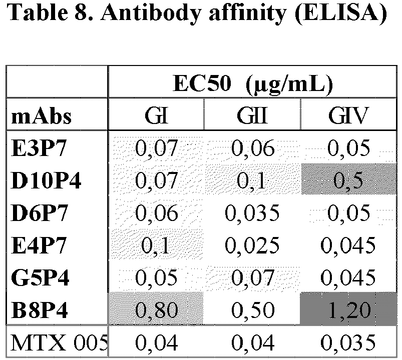

**Table 9. Antibody lineage features**

| | **HEAVY CHAIN** | | | | **LIGHT CHAIN** | | | |
|---|---|---|---|---|---|---|---|---|
| | **V gene** | **% Identity V gene** | **J gene** | **% Identity J gene** | **V gene** | **% Identity V gene** | **J gene** | **% Identity J gene** |
| **E3P7** | IGHV3-23 | 93,4 | IGHJ5 | 86,27 | IGKV3-20 | 97,5 | IGJKJ2 | 97.22 |
| **D10P4** | IGHV3-23 | 92,0 | IGHJ5 | 88.24 | IGKV3-20 | 97,2 | IGJKJ2 | 80.56 |
| **D6P7** | IGHV3-23 | 93,4 | IGHJ5 | 88.24 | IGKV3-20 | 95,4 | IGJKJ2 | 97.22 |
| **E4P7** | IGHV3-23 | 94,1 | IGHJ5 | 88,24 | IGKV3-20 | 96,8 | IGJKJ2 | 97.22 |
| **G5P4** | IGHV3-23 | 91,3 | IGHJ5 | 86.27 | IGKV3-20 | 98,2 | IGJKJ2 | 97.22 |
| **B8P4** | IGHV3-23 | 92,7 | IGHJ5 | 86.27 | IGKV3-20 | 97,2 | IGJKJ2 | 97.22 |

High affinity for VP1 was further confirmed by BLI, revealing K_{D} in the nanomolar to sub-nanomolar range. The monoclonal antibody E3P7 showed a remarkably high affinity, with a K_{D} of 0.09 nm, 0.24 nm and 0.3 nm for VP1 pentamer from genotypes I, II, and IV, respectively. It was followed by the D10P4 antibody, with an affinity of 1.40 nm and 1.77 nm for the genotypes I and II. In contrast, antibody MTX005 displayed an affinity of 17.94 nm, 17.07 nm and 23.79 nm for genotypes I, II and IV.

**Table 10. Antibody affinity (BLI)**

| Index | **KD (nM)** | ka (1/Ms) | kdis (1/s) |
|---|---|---|---|
| **Genotype I** | | | |
| **E3P7** | 0,09 | 1,27E+05 | 1,18E-05 |
| **D10P4** | 1,40 | 9,08E+04 | 1,27E-04 |
| **D6P7** | 7,78 | 6,02E+04 | 4,68E-04 |
| **E4P7** | 2,30 | 9,46E+04 | 2,12E-04 |
| **G5P4** | 5,58 | 7,42E+04 | 4,13E-04 |
| **MTX 005** | 17,94 | 1,10E+05 | 1,95E-03 |

| **Genotype II** | | | |
|---|---|---|---|
| **E3P7** | 0,24 | 1,14E+05 | 2,73E-05 |
| **D10P4** | 5,77 | 9,19E+04 | 5,31E-04 |
| **D6P7** | 4,51 | 9,82E+04 | 4,43E-04 |
| **E4P7** | 1,02 | 1,69E+05 | 1,71E-04 |
| **G5P4** | 27,97 | 7,33E+04 | 2,04E-03 |
| **MTX 005** | 17,07 | 2,16E+05 | 3,63E-03 |

| **Genotype IV** | | | |
|---|---|---|---|
| **E3P7** | 0,298 | 1,02E+05 | 3,14E-05 |
| **D10P4** | 104,81 | 6,58E+04 | 6,25E-03 |
| **D6P7** | 32,14 | 8,07E+04 | 2,46E-03 |
| **E4P7** | 4,91 | 1,06E+05 | 5,17E-04 |
| **G5P4** | 136,32 | 5,29E+04 | 6,38E-03 |
| **MTX 005** | 23,79 | 1,90E+05 | 4,45E-03 |

To increase half-life of antibodies, an LS mutation was introduced into antibodies E4P7, D10P4, D6P7 and E3P7: this mutation did not impact the affinity of antibodies to VP1 pentamer (data not shown).

### 2.4. Antibody neutralizing activity

The neutralizing activity of the 6 monoclonal antibodies was evaluated with a neutralization assay as described above. The potency of each monoclonal antibody was measured as IC₅₀ **(Tables 11 to 13).**

Overall, all monoclonal antibodies neutralized the three BKV genotypes with high potency, except B8P4, which neutralized genotype IV only at a higher concentration. Monoclonal antibodies E3P7 and D10P4 exhibited the highest potency across all the 3 genotypes, neutralizing genotype I and II in the sub-ng/ml range, and genotype IV in the ng/ml range. The potency of E3P7 and D10P4 were actually comparable to the potency of the MTX005 antibody, and far higher than the potency of the P8D11 antibody.

**Table 11. Antibody potency (BKV pseudovirions)**

| | **IC50 (ng/mL)** | | |
|---|---|---|---|
| **mAbs** | **GI** | **GII** | **GIV** |
| **E3P7** | 0.14 | 0.11 | 0.48 |
| **D10P4** | 0.14 | 0.18 | 2.62 |
| **D6P7** | 0.45 | 0.20 | 3.22 |
| **E4P7** | 0.57 | 0.13 | 1.88 |
| **G5P4** | 0.20 | 0.15 | 5.23 |
| **B8P4** | 0.13 | 4.95 | 54.49 |
| **MTX 005** | 0.03 | 0.08 | 0.39 |
| **MTX 005 variant** | 0.09 | 0.11 | 0.50 |
| **P8D11** | 4.22 | 1.45 | 4.72 |

The neutralizing activity of antibodies D10P4 and E3P7 was further confirmed using fully replicative BKV and MRC5 cells as targets **(Table 12).**

**Table 12. Antibody potency (fully replicative BKV virions)**

| | **BKV genotypes** | | |
|---|---|---|---|
| **Mabs IC50 (ng/mL)** | **I** | **II** | **IV** |
| **D10P4** | 0.06 | 0.05 | 0.66 |
| **E3P7** | 0.03 | 0.005 | 0.18 |
| **319 C07 wild type** | 0.05 | 0.03 | 0.38 |
| **P8D11** | 3.28 | 14.92 | 1.65 |

To determine whether these two antibodies targeted a conserved epitope across human polyomaviruses, their neutralizing activity was also evaluated against JCV **(Table 13).** Interestingly, both E3P7 and D10P4 neutralized JCV, with an IC50 in the sub-µg/ml range for the E3P7 antibody. In contrast, antibody MTX005 totally lacked the capacity to neutralize JCV.

**Table 13. Antibody potency (JCV pseudovirions)**

| **Mabs IC50 (ng/mL)** | **JCV-PsV** |
|---|---|
| **D10P4** | 1268 |
| **E3P7** | 118 |
| **319C07 Wild type** | Negative |

At last, seven mutations commonly found in transplant patients were introduced into VP1 to assess the neutralization activity of D10P4 and E3P7 across these polymorphisms. Both antibodies strongly neutralized all BKV mutants, confirming their broad spectrum of activity. Antibodies D10P4 and E3P7 significantly outperformed MTX005 (i.e. 319C07 wt) with regard to neutralization of the BKV double-mutant A72V/E73Q, which is among the most frequently observed mutations in kidney transplant recipients experiencing high BKV replication.

**Table 14. Ratio increase of neutralization titers of BKV mutants to BKV wild-type**

| | **Mutations in BKV Genotype I** | | | | | | **Mutations in BKV genotype IV** |
|---|---|---|---|---|---|---|---|
| **Ratio increase of Mab IC50** | **D60N** | **L68Q** | **A72E73** | **I E73Q** | **I D75N** | **I E82D** | **IV D77Q** |
| **D10P4** | 1.48 | 1.25 | 3.61 | 0.79 | 0.49 | 0.48 | 1.35 |
| **E3P7** | 7.05 | 0.30 | 3.13 | 7.12 | 9.99 | 1.40 | 4.83 |
| **319C07 variant** | 5.94 | 1.59 | 1858 | 8.56 | 12.63 | 4.48 | 5.99 |
| **319C07 wild type** | 4.38 | 0.90 | 123 | 1.77 | 2.22 | 0.77 | 1.72 |

Similar experiments were conducted with LS mutated antibodies. This mutation did not impact the neutralization capacity of antibodies D10P4 and E3P7 (data not shown).

### 2.5. Antibody auto and polyreactivity

Because antibody autoreactivity can be an obstacle to translating a candidate antibody into a clinical product, autoreactivity of the most potent antibodies (D10P4 and E3P7) was evaluated on HEp-2 cells. No reactivity was observed (data not shown), confirming the absence of autoreactivity of the antibodies of the invention. No reactivity was observed either in the ELISA assay. In contrast, antibody MTX005 uses the VH4-34 gene segment which contains a known autoreactive motif (Sabouri Z., et al.; 2014; Proc Natl Acad Set USA ; 111(25): E2567-2575).

### 2.6. Antibody epitopes

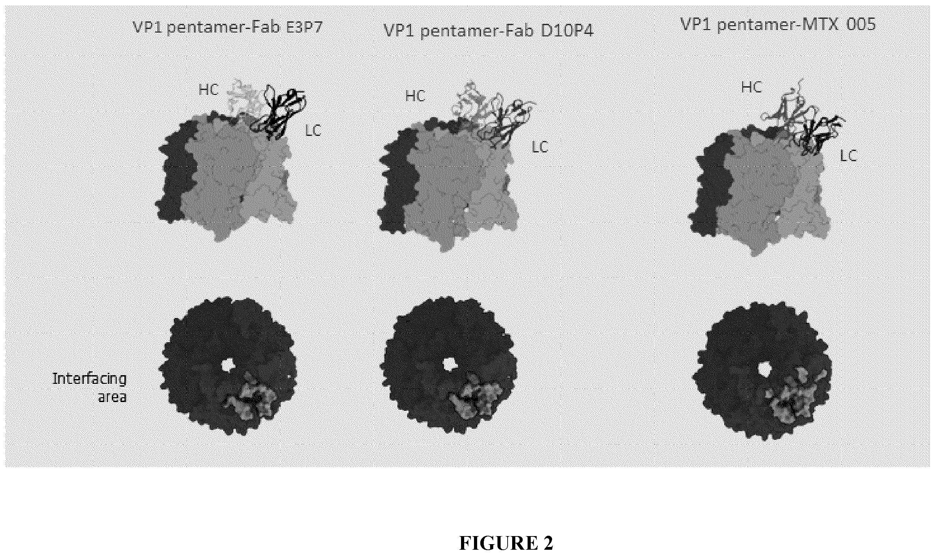

The two most potent monoclonal antibodies D10P4 and E3P7 were selected for epitope determination using cryo-EM. To do so, Fabs of these two antibodies were produced and complexed with recombinant VP1 pentamers and/or VLPs for study by cryo-EM. As expected, both antibodies bound VP1 in a similar manner, at the same angle, primarily targeting the BC loop **(****Figure 2****).**

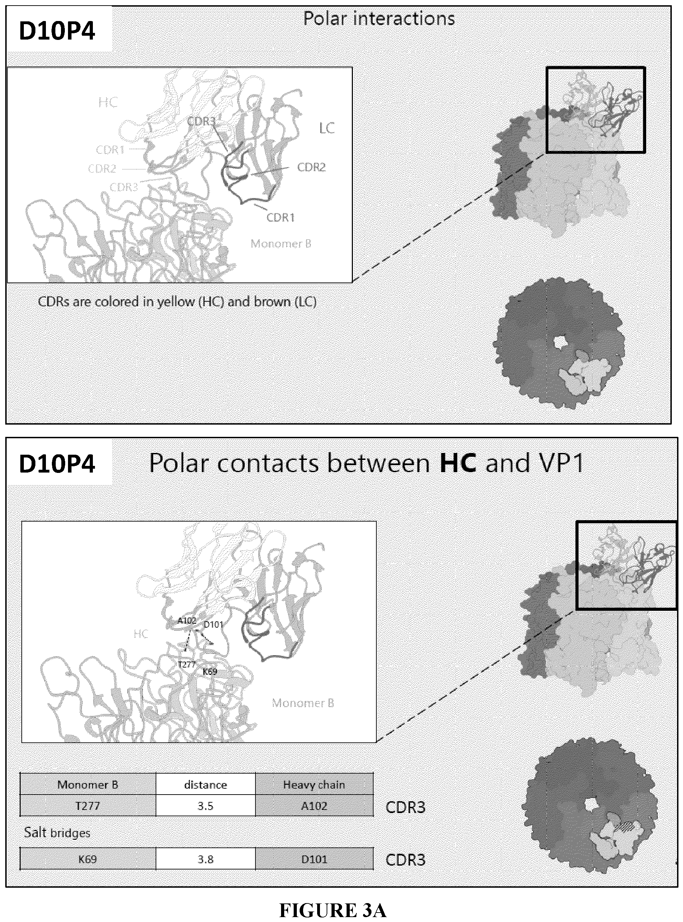

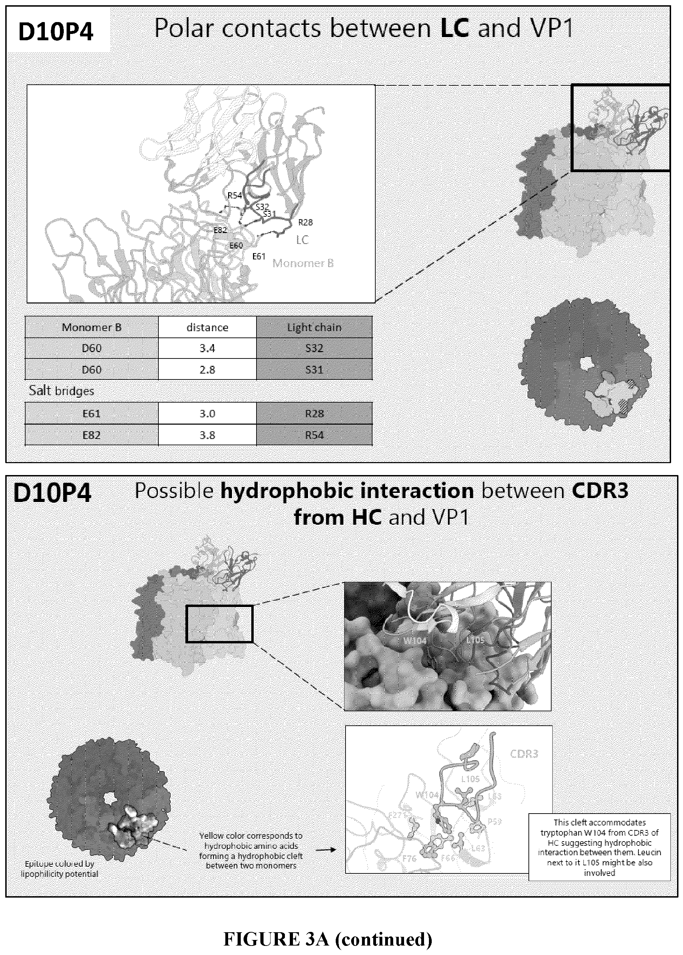

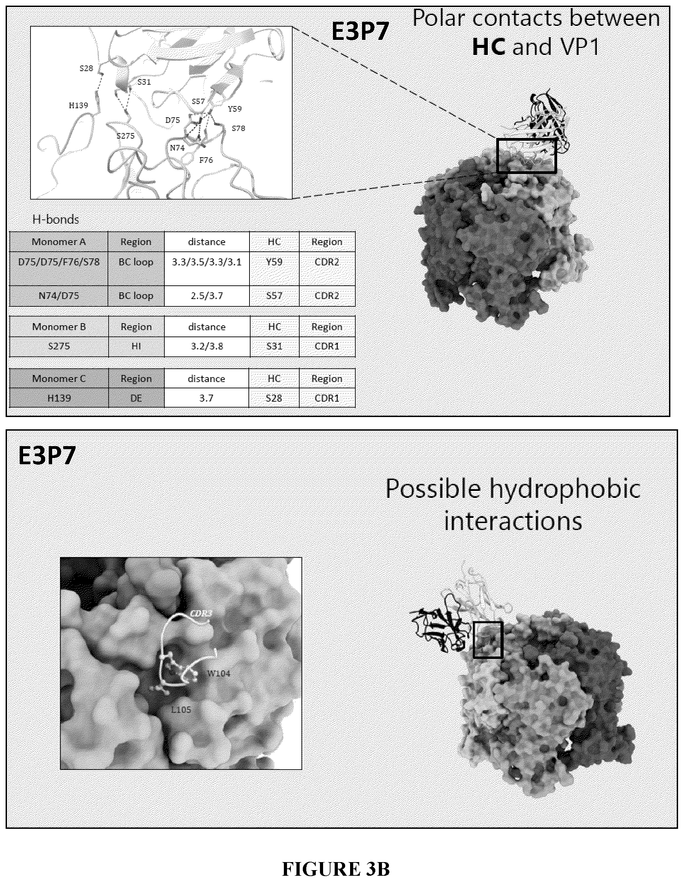

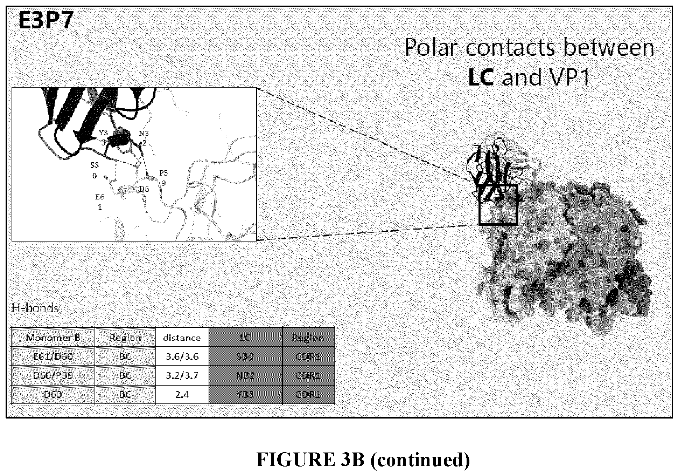

Details of interactions between antibodies and VP 1 are shown in **Figure 3****,** as well as in **Table 15** below. While their interfacing areas on the VP1 pentamers were similarly encompassing three VP1 monomers, these 2 antibodies differed in their fine interactions. E3P7 displayed more contact amino acid residues, and notably did not interact with VP1 through its HCDR3.

**Table 15. D10P4 and E3P7 epitopes as determined by cryo-EM**

| **VP1** | | | **Fab** | | | | |
|---|---|---|---|---|---|---|---|
| **VP1 pentamer residue** | **Monomer involved** | **VP1 loop** | **Anti-VPI Fab residue** | **VP1_Fab distance** | **Fab chain** | **Interactions** | **Fab region** |
| **VLP_Fab D10P4** | | | | | | | |
| D60 | B | BC | S31 | 3,4 | LC | polar | CDR1 |
| D60 | B | BC | S32 | 2,8 | LC | polar | CDR1 |
| E61 | B | BC | R28 | 3,0 | LC | salt bridge | CDR1 |
| K69 | B | BC | D101 | 3,8 | HC | salt bridge | CDR3 |
| E82 | B | BC | R54 | 3,8 | LC | salt bridge | FR3 |
| T277 | B | HI | A102 | 3,5 | HC | polar | CDR3 |

| **VLP_Fab E3P7** | | | | | | | |
|---|---|---|---|---|---|---|---|
| P59 | B | BC | N32 | 3,7 | LC | H-bond | CDR1 |
| D60 | B | BC | S30 | 3,6 | LC | H-bond | CDR1 |
| D60 | B | BC | N32 | 3,2 | LC | H-bond | CDR1 |
| D60 | B | BC | Y33 | 2,4 | LC | H-bond | CDR1 |
| E61 | B | BC | S30 | 3,6 | LC | H-bond | CDR1 |
| N74 | A | BC | S57 | 2,5 | HC | H-bond | CDR2 |
| D75 | A | BC | Y59 | 3,3/3,5 | HC | H-bond | CDR2 |
| D75 | A | BC | S57 | 3,7 | HC | H-bond | CDR2 |
| F76 | A | BC | Y59 | 3,3 | HC | H-bond | CDR2 |
| S78 | A | BC | Y59 | 3,1 | HC | H-bond | CDR2 |
| H139 | C | DE | S28 | 3,7 | HC | H-bond | CDR1 |
| S275 | B | HI | S31 | 3,2/3,8 | HC | H-bond | CDR1 |

| **VLP geno I pentamer_Fab MT 319C07 (MTX 005)** | | | | | | | |
|---|---|---|---|---|---|---|---|
| D60 | B | BC | S32 | 3,7 | LC | H-bond | CDR1 |
| N62 | B | BC | Y33 | 3,6 | LC | H-bond | CDR1 |
| E73 | A | BC | T101 | 3,3 | HC | H-bond | CDR3 |
| F76 | A | BC | S102 | 3,3 | HC | H-bond | CDR3 |
| S80 | B | BC | D61 | 3,6/3,2 | LC | H-bond | LFR3 |
| K84 | B | BC | S53 | 3,4 | LC | H-bond | CDR2 |
| H139 | C | DE | Y110 | 2,8 | HC | H-bond | CDR3 |
| T277 | B | HI | D106 | 3,0 | HC | H-bond | CDR3 |

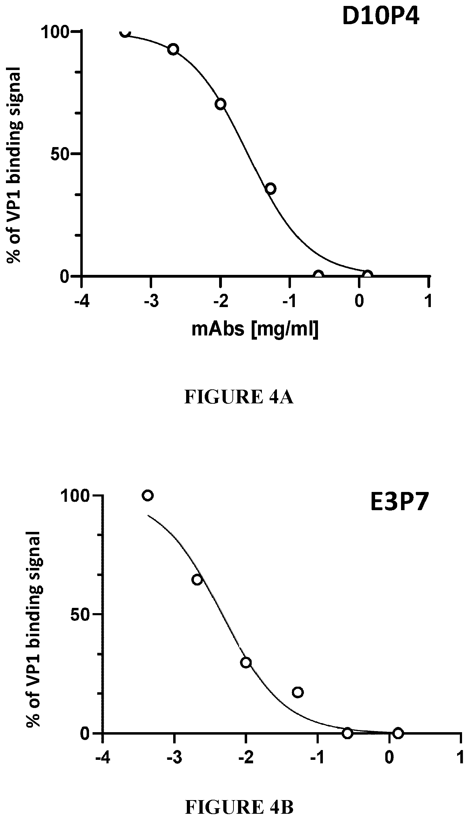

Because epitope mapping strongly suggested that antibodies D10P4 and E3P7 neutralized BKV by blocking access to the cellular receptor, the ability of these two most potent antibodies to block the binding of VP1 pentamers to BKV receptor-expressing HEK293 cells was assessed. As expected, both antibodies inhibited the binding of VP1 to target cells in a dose-dependent manner, confirming a mechanism of action through receptor binding inhibition **(****Figure 4****).**

### 3. CONCLUSION

We describe herein the generation of a panel of six BKV neutralizing human mAbs obtained from peripheral memory B cells of a kidney transplant patient. These monoclonal antibodies bound to the BKV VP1 capsid protein with a high affinity, and potently cross-neutralized the various genotypes of BKV, as well as JCV, providing strong leads for the development of therapeutic and preventive approach of BKV reactivation in transplant patients. These antibodies are also suitable for diagnostic purpose of a BKV and/or JCV infection.

## Claims

1. A monoclonal antibody, preferably a human monoclonal antibody, or antigen-binding fragment or chain thereof, that binds and neutralizes a plurality of BK virus subtypes including at least subtypes I, II and IV, said antibody having a combination of a heavy chain variable domain (VH) and/or a light chain variable domain (VL) with the six following complementary-determining regions (CDRs):
• a VH-CDR1 comprising, or consisting of, amino acid sequence GFX₁X₂RX₃YX₄ (SEQ ID NO: 1), wherein X₁ is S, T or N, preferably is S; X₂ is F, I or L, preferably is F or I; X₃ is S or G; and X₄ is A or G, preferably is A;
• a VH-CDR2 comprising, or consisting of, amino acid sequence LX₅SX₆GX₇X₈X₉, wherein X₅ is N, T, S or R, preferably is N or T; X₆ is A, S or N, preferably is A or N; X₇ is V, A or T, preferably is V or A; X₈ is S or T; X₉ is T or A, preferably is T;
• a VH-CDR3 comprising, or consisting of, amino acid sequence AX₁₀DRX₁₁X₁₂X₁₃WLGSEPX₁₄DP (SEQ ID NO: 2), wherein X₁₀ is K or R, preferably is K; X₁₁ is G, D or N, preferably is G or D; X₁₂ is V or A; X₁₃ is E or Q, preferably is E; X₁₄ is I, F or L, preferably is I or F;
• a VL-CDR1 comprising, or consisting of, amino acid sequence QX₁₅X₁₆X₁₇X₁₈X₁₉Y, wherein X₁₅ is R or S, preferably is R; X₁₆ is V or L, preferably is V; X₁₇ is S or G, preferably is S; X₁₈ is S or N, preferably is S; X₁₉ is N or S;
• a VL-CDR2 comprising, or consisting of, amino acid sequence X₂₀AS, wherein X₂₀ is R, G or D, preferably is R or G; and
• a VL-CDR3 comprising, or consisting of, amino acid sequence QQYGSX₂₁PRS (SEQ ID NO: 3), wherein X₂₁ is S or P, preferably is S.

2. The antibody, or antigen-binding fragment thereof, according to claim 1, wherein:
• the VH-CDR1 comprises, or consists of, amino acid sequence SEQ ID NO: 6, 13, 19, 25, 28 or 34;
• the VH-CDR2 comprises, or consists of, amino acid sequence SEQ ID NO: 7, 14, 20, 29 or 35;
• the VH-CDR3 comprises, or consists of, amino acid sequence SEQ ID NO: 8, 15, 30 or 36;
• the VL-CDR1 comprises, or consists of, amino acid sequence SEQ ID NO: 9, 16, 21, 31 or 37;
• the VL-CDR2 comprises, or consists of, amino acid sequence RAS, GAS, or DAS; and
• the VL-CDR3 comprises, or consists of, amino acid sequence SEQ ID NO: 10 or 22.

3. The antibody, or antigen-binding fragment thereof, according to claim 1 or 2, wherein the six complementary determining regions (CDRs) are selected from either of the following combinations:
(a) VH-CDR1, VH-CDR2 and VH-CDR3 comprising, or consisting of, amino acid sequence SEQ ID NO: 6, 7 and 8, respectively; and VL-CDR1, VL-CDR2 and VL-CDR3 comprising, or consisting of, amino acid sequence SEQ ID NO: 9, RAS and SEQ ID NO: 10, respectively; or
(b) VH-CDR1, VH-CDR2 and VH-CDR3 comprising, or consisting of, amino acid sequence SEQ ID NO: 13, 14 and 15, respectively; and VL-CDR1, VL-CDR2 and VL-CDR3 comprising, or consisting of, amino acid sequence SEQ ID NO: 16, GAS and SEQ ID NO: 10, respectively; or
(c) VH-CDR1, VH-CDR2 and VH-CDR3 comprising, or consisting of, amino acid sequence SEQ ID NO: 19, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(d) VH-CDR1, VH-CDR2 and VH-CDR3 comprising, or consisting of, amino acid sequence SEQ ID NO: 25, 20 and 15, respectively; and VL-CDR1, VL-CDR2 and VL-CDR3 comprising, or consisting of, amino acid sequence SEQ ID NO: 21, DAS and SEQ ID NO: 22, respectively; or
(e) VH-CDR1, VH-CDR2 and VH-CDR3 comprising, or consisting of, amino acid sequence SEQ ID NO: 28, 29 and 30, respectively; and VL-CDR1, VL-CDR2 and VL-CDR3 comprising, or consisting of, amino acid sequence SEQ ID NO: 31, GAS and SEQ ID NO: 10, respectively; or
(f) VH-CDR1, VH-CDR2 and VH-CDR3 comprising, or consisting of, amino acid sequence SEQ ID NO: 34, 35 and 36, respectively; and VL-CDR1, VL-CDR2 and VL-CDR3 comprising, or consisting of, amino acid sequence SEQ ID NO: 37, GAS and SEQ ID NO: 10, respectively.

4. The antibody, or antigen-binding fragment thereof, according to any one of the preceding claims, in which:
• the heavy chain variable domain (VH) comprises, or consists of, an amino acid sequence having at least 80% sequence identity to : EVQLLESGGX₂₂LVQPGGSLRLSCX₂₃ASGFX₁X₂RX₃YX₄MNWVRQAPG X₂₄GLEWVSSLX₅SX₆GX₇X₈X₉YYAX₂₅SVKGRFTISRDNX₂₆KNTX₂₇X₂₈L QMX₂₉SLRGEDTAVYYCAX₁₀DRX₁₁X₁₂X₁₃WLGSEPX₁₄DPWGQGTX₃₀VT VSS (SEQ ID NO: 4), wherein X₁ to X₁₄ are as defined in claim 1; X₂₂ is G or A, preferably is G; X₂₃ is A or V; X₂₄ is K or R, preferably is K; X₂₅ is E or D; X₂₆ is S or F, preferably is S; X₂₇ is L or M, preferably is L; X₂₈ is Y or F; X₂₉ is N, H or D, preferably is N or H; and X₃₀ is L or M, preferably is L; and
• the light chain variable domain (VL) comprises, or consists of, an amino acid sequence having at least 80 % sequence identity to: EIVLTQSPGTLSLSPGERATLSCRASQX₁₅X₁₆X₁₇X₁₈X₁₉YLAWYQHKX₃₁G X₃₂APRLLIYX₂₀ASX₃₃RAX₃₄GIPDRFSGSX₃₅SGTDFTLTISRLEPEDFAVY YCQQYGSX₂₁PRSFGQGTKLEIK (SEQ ID NO: 5), wherein X₁₅ to X₂₁ are as defined in claim 1; X₃₁ is P or F; X₃₂ is Q or L, preferably is Q; X₃₃ is S or R; X₃₄ is T or I, preferably is T; and X₃₅ is G or E, preferably is G;
with the proviso that the CDRs are as defined in any one of claims 1 to 3.

5. The antibody, or antigen-binding fragment thereof, according to any one of the preceding claims, wherein :
• the VH comprises, or consists of, an amino acid sequence having at least 80% sequence identity to any one of SEQ ID NO: 11, 17, 23, 26, 32 or 38, preferably SEQ ID NO: 11 or 17; and
• the VL comprises, or consists of, an amino acid sequence having at least 80% sequence identity to any one of SEQ ID NO: 12, 18, 24, 27, 33 or 39, preferably SEQ ID NO: 12 or 18;
with the proviso that the CDRs are as defined in claim 2 or 3.

6. The antibody, or antigen-binding fragment thereof, according to any one of the preceding claims, wherein the VH and the VL are selected from any one of the following combinations:
(i) VH having at least 80% sequence identity to SEQ ID NO: 11 and VL having at least 80% sequence identity to SEQ ID NO: 12, with the proviso that the CDRs thereof are as defined in claim 3(a); or
(ii) VH having at least 80% sequence identity to SEQ ID NO: 17 and VL having at least 80% sequence identity to SEQ ID NO: 18, with the proviso that the CDRs thereof are as defined in claim 3(b); or
(iii) VH having at least 80% sequence identity to SEQ ID NO: 23 and VL having at least 80% sequence identity to SEQ ID NO: 24, with the proviso that the CDRs thereof are as defined in claim 3(c); or
(iv) VH having at least 80% sequence identity to SEQ ID NO: 26 and VL having at least 80% sequence identity to SEQ ID NO: 27, with the proviso that the CDRs thereof are as defined in claim 3(d); or
(v) VH having at least 80% sequence identity to SEQ ID NO: 32 and VL having at least 80% sequence identity to SEQ ID NO: 33, with the proviso that the CDRs thereof are as defined in claim 3(e); or
(vi) VH having at least 80% sequence identity to SEQ ID NO: 38 and VL having at least 80% sequence identity to SEQ ID NO: 39, with the proviso that the CDRs thereof are as defined in claim 3(f).

7. The antibody, or antigen-binding fragment thereof, according to any one of the preceding claims, that further binds and neutralizes JC virus (JCV).

8. An antibody, or antigen-binding fragment thereof, that binds and neutralizes a plurality of BK virus subtypes including at least subtype I, II and IV, wherein said antibody has one or more of the following characteristics:
(1) competes for binding to a BKV viral protein 1 (VP1), with a reference antibody as defined in claim 3 or 6, such as antibody E3P7 (VH: SEQ ID NO: 11; VL: SEQ ID NO: 12), D10P4 (VH: SEQ ID NO: 17; VL: SEQ ID NO: 18), D6P7 (VH: SEQ ID NO: 23; VL: SEQ ID NO: 24), E4P7(VH: SEQ ID NO: 26; VL: SEQ ID NO: 27), G5P4 (VH: SEQ ID NO: 32; VL: SEQ ID NO: 33), and/or B8P4 (VH: SEQ ID NO: 38; VL: SEQ ID NO: 39); and/or
(2) binds to an epitope comprising one or more amino acids selected from P59, D60, E61, L63, F66, L68, K69, N74, D75, F76, S78, E82, H139, F271, T277 and/or S275 in a BKV viral protein 1 (VP1) of SEQ ID NO: 40; and/or
(3) has reduced binding or loses binding to a mutant BKV viral protein 1 (VP1), said mutant containing one or more of the following amino acid mutations in SEQ ID NO: 40: P59A, D60A, E61A, L63A, F66A, L68A, K69A, N74A, D75A, F76A, S78A, E82A, H139A, F271A, T277A and/or S275A.

9. The antibody, or antigen-binding fragment thereof, according to any one of the preceding claims, which is an IgG, an IgA, an sIgA, an IgM, or a nanobody, preferably is an IgG, said IgG being preferably an IgG1, IgG2, IgG3 or IgG4.

10. The antibody, or antigen-binding fragment thereof, according to any one of the preceding claims, further comprising, especially in the Fc region, modifications which modulate Fc/FcRn binding, complement binding, complement-dependent cytotoxicity (CDC), antibody-dependent cellular toxicity (ADCC), antibody-dependent cell phagocytosis (ADCP), glycosylation and/or pharmacokinetics such as half-life.

11. The antibody, or antigen-binding fragment thereof, according to any one of the preceding claims, which comprises one or more non-natural amino acids.

12. The antibody, or antigen-binding fragment thereof, according to any one of the preceding claims, which is comprised in an immunoconjugate, such as an immunoconjugate wherein the antibody or antigen-binding fragment is conjugated with another moiety, such as another moiety selected from another antibody, including another anti-BKV antibody, a cytotoxic moiety (to form an ADC), a cell-penetrating compound or a tissue-penetrating compound.

13. The antibody, or antigen-binding fragment thereof, according to any one of claims 1 to 12, which is labeled with a detectable molecule.

14. A combination of two or more antibodies, or antigen-binding fragments thereof, as defined in any one of claims 1 to 13.

15. A set of nucleic acids encoding the antibody, or antigen-binding fragment thereof, as defined in any one of claims 1 to 13, or a vector comprising said set of nucleic acids.

16. A host cell expressing the antibody, or antigen-binding fragment thereof, as defined in any one of claims 1 to 13, or comprising the set of nucleic acids or the vector as defined in claim 15.

17. A pharmaceutical or diagnostic composition comprising:
(1) at least one of the antibody, or antigen-binding fragment thereof, as defined in any one of claims 1 to 13, or any combination thereof; and
(2) optionally at least one pharmaceutically or diagnostically acceptable excipient.

18. The pharmaceutical composition according to claim 17, which in a form suitable for a sustainable release, and/or for intravenous, intramuscular, subcutaneous, intranasal, or intrathecal administration, or for administration by infusion or by aerosol.

19. A pharmaceutical composition as defined in claim 17 or 18, for use as a medicament.

20. The pharmaceutical composition for use according to claim 19, in the treatment of a BK virus (BKV) infection or associated disorder thereof in a subject in need thereof.

21. The pharmaceutical composition for use according to claim 20, wherein the subject is an immunocompromised subject, especially a graft-recipient such as a kidney-graft or bone-marrow-graft recipient.

22. The pharmaceutical composition for use according to claim 20 or 21, wherein the disorder associated with a BK virus (BKV) infection is a BKV-related leukodystrophy.

23. A pharmaceutical composition as defined in claim 17 or 18, and at least one antibody reacting with a non-BKV virus, as a combined preparation for simultaneous, separate or sequential use as a medicament, the non-BKV virus being selected from the group consisting of a SARS-CoV-2 virus and other coronaviruses, a Respiratory Syncytial Virus (RSV), a Herpes Virus Simplex (HSV), an Epstein-Barr virus (EBV), a cytomegalovirus (CMV), an influenza virus, a parainfluenza virus, a metapneumovirus, and a JC virus.

24. Two or more antibodies or antigen-binding fragments thereof as defined in any one of claims 1 to 13, as a combined preparation for simultaneous, separate or sequential use as a medicament, preferably in the treatment of a BK virus (BKV) infection or associated disorder thereof, in a subject in need thereof.

25. An *in vitro* use of the diagnostic composition as defined in claim 17, for detecting the presence of BKV in a sample, preferably in a biological sample.

26. An *in vitro* use of an anti-idiotypic antibody that binds to the antibody or antigen-binding fragment thereof as defined in any one of claims 1 to 13, for measuring the concentration of said antibody or fragment in a sample, preferably in a biological sample.
